(19) Europäisches Patentamt
European Patent Office
Office européen des brevets

(11) EP 4 722 262 A1

(12) EUROPEAN PATENT APPLICATION
published in accordance with Art. 153(4) EPC

(43) Date of publication:
08.04.2026 Bulletin 2026/15

(21) Application number: 24836108.1

(22) Date of filing: 04.07.2024

(51) International Patent Classification (IPC):
*C08F 220/10* (2006.01) *A61K 6/887* (2020.01)
*A61K 6/896* (2020.01) *B33Y 70/00* (2020.01)
*B33Y 80/00* (2015.01) *C08F 2/44* (2006.01)
*C08F 290/06* (2006.01)

(52) Cooperative Patent Classification (CPC):
**A61K 6/887; A61K 6/896; B33Y 70/00; B33Y 80/00; C08F 2/44; C08F 220/10; C08F 290/06**

(86) International application number:
PCT/JP2024/024289

(87) International publication number:
WO 2025/009596 (09.01.2025 Gazette 2025/02)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 05.07.2023 JP 2023111074

(71) Applicant: Mitsui Chemicals, Inc.
Tokyo 104-0028 (JP)

(72) Inventors:
- ENDO, Suguru
Sodegaura-shi, Chiba 299-0265 (JP)
- CHOI, Dongil
Sodegaura-shi, Chiba 299-0265 (JP)
- SAKAMAKI, Toshikazu
Sodegaura-shi, Chiba 299-0265 (JP)

(74) Representative: Hoffmann Eitle
Patent- und Rechtsanwälte PartmbB
Arabellastraße 30
81925 München (DE)

# (54) PHOTOCURABLE COMPOSITION, THREE-DIMENSIONAL SHAPED OBJECT, CASTING MOLD, METHOD FOR PRODUCING CURED OBJECT, AND METHOD FOR PRODUCING DENTAL PROSTHESIS

(57) A photocurable composition includes: a (meth)acrylic monomer (A) having one or more (meth)acryloyl groups and not having a siloxane bond; a (meth)acrylic monomer (B) having two or more (meth)acryloyl groups and a siloxane bond; a compound (C) not having a (meth)acryloyl group and having a solubility parameter (SP value) of from 7.00 to 15.00 $(cal/cm^3)^{1/2}$; and a photopolymerization initiator.

Fig. 1

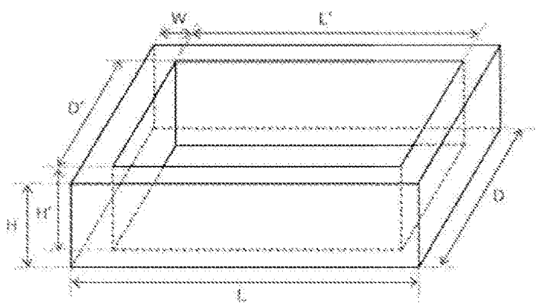


EP 4 722 262 A1

## Description

Technical Field

The present disclosure relates to a photocurable composition, a three-dimensional object, a casting mold, a method for manufacturing a cured article, and a method for manufacturing a dental prosthesis.

Background Art

In recent years, studies have been conducted regarding dental products such as dental prostheses and instruments used in the oral cavity. For example, from the viewpoint of efficiency in shaping these dental products, a method for manufacturing a three-dimensional object such as dental products by stereolithography using a 3D printer is known (for example, refer to Japanese Patent No. 4160311).

SUMMARY OF INVENTION

Technical Problem

Meanwhile, a method for producing dental prostheses such as denture with a base without using stereolithography using a 3D printer or the like has conventionally existed.

According to this method, for example, in the case of manufacturing a dental prosthesis such as a denture with a base, first, a mold of the oral cavity directly from a patient is acquired using an impression material, and a model simulating the oral cavity of the patient is produced with reference to the impression material. A mold is taken from the model using silicone. The molded object is used as a casting mold, into which a curable composition is injected, and a dental prosthesis such as a denture with a base can be manufactured by room-temperature polymerization.

According to this conventional method that does not use stereolithography, as described above, there is a problem in that the steps such as producing a model simulating the oral cavity of the patient and taking a mold using silicone are complicated.

In contrast, according to a method for directly manufacturing a denture with a base by stereolithography using a 3D printer, the above-mentioned complexity is eliminated. However, compared to the conventional method that does not use a 3D printer, the materials that can be appropriately cured by stereolithography are very limited, and therefore, the characteristics of the obtained dental prosthesis such as a denture with a base also become limited. For example, in the conventional method, room-temperature polymerization is used with a casting mold, and in this method, many materials that can improve aesthetic properties or improve physical properties can be used. In contrast, materials used in such room-temperature polymerization are often not usable in stereolithography by a 3D printer. As a result, in dental prostheses such as dentures with a base obtained by stereolithography using a 3D printer, it becomes difficult to make adjustments to obtain desired properties.

As described above, there is a demand for a method for manufacturing a dental prosthesis that allows for simple production of the dental prosthesis and also facilitates adjustment to obtain desired properties.

In order to achieve the above object, the present inventors have found a method in which a casting mold for manufacturing a dental prosthesis is manufactured by stereolithography using a 3D printer, and the dental prosthesis is manufactured using the casting mold.

In the case where a casting mold for a dental prosthesis is produced using a photocurable composition and the dental prosthesis is manufactured using the casting mold, it is desirable to improve the releasability of the dental prosthesis from the casting mold. From the viewpoint of improving the releasability of the dental prosthesis from the casting mold, the present inventors have found that a photocurable composition containing a (meth)acrylic monomer component having a siloxane bond can be used. However, using a (meth)acrylic monomer component having a siloxane bond may cause the surface of a three-dimensional object such as a casting mold to become rough, and, for example, from the viewpoint of the appearance of the three-dimensional object, it is desirable to reduce the surface roughness.

An object of one aspect of the disclosure is to provide a photocurable composition capable of producing a three-dimensional object in which surface roughness is suppressed, as well as a three-dimensional object using the photocurable composition, a casting mold, a method for manufacturing a cured article, and a method for manufacturing a dental prosthesis. Solution to Problem

Means for solving the above problems include the following aspects.

<1> A photocurable composition, including:

a (meth)acrylic monomer (A) having one or more (meth)acryloyl groups and not having a siloxane bond;

a (meth)acrylic monomer (B) having two or more (meth)acryloyl groups and a siloxane bond;
a compound (C) not having a (meth)acryloyl group and having a solubility parameter (SP value) of from 7.00 to 15.00 $(cal/cm^3)^{1/2}$; and
a photopolymerization initiator.

<2> The photocurable composition according to <1>, in which an absolute value of a difference between an average value of solubility parameters (SP values) of (meth)acrylic monomer components contained in the photocurable composition and an average value of the solubility parameter (SP value) of the compound (C) contained in the photocurable composition is 4.00 $(cal/cm^3)^{1/2}$ or less.
<3> The photocurable composition according to <1> or <2>, in which a cohesive energy density (cal/mol) of the compound (C) is 10,000 or more.
<4> The photocurable composition according to any one of <1> to <3>, in which
the (meth)acrylic monomer (A) contains:

a di(meth)acrylic monomer (A-1) having at least one of a ring structure or a urethane bond, and two (meth) acryloyloxy groups, and not having a siloxane bond; and
a mono(meth)acrylic monomer (A-2) having one (meth)acryloyloxy group and an aromatic ring, and not having a siloxane bond.

<5> The photocurable composition according to <4>, in which a molecular weight of the di(meth)acrylic monomer (A-1) is from 400 to 8,000.
<6> The photocurable composition according to <4> or <5>, in which a molecular weight of the mono(meth)acrylic monomer (A-2) is from 160 to 400.
<7> The photocurable composition according to any one of <1> to <6>, in which a molecular weight of the (meth) acrylic monomer (B) is from 400 to 5,000.
<8> The photocurable composition according to any one of <4> to <7>, in which a content of the di(meth)acrylic monomer (A-1) contained in the photocurable composition is from 3% by mass to 50% by mass with respect to a total content of (meth)acrylic monomer components and the compound (C) contained in the photocurable composition.
<9> The photocurable composition according to any one of <4> to <8>, in which a content of the mono(meth)acrylic monomer (A-2) contained in the photocurable composition is from 20% by mass to 85% by mass with respect to a total content of (meth)acrylic monomer components and the compound (C) contained in the photocurable composition.
<10> The photocurable composition according to any one of <1> to <9>, in which a content of the (meth)acrylic monomer (B) contained in the photocurable composition is from 1% by mass to 35% by mass with respect to a total content of (meth)acrylic monomer components and the compound (C) contained in the photocurable composition.
<11> The photocurable composition according to any one of <1> to <10>, in which a content of the compound (C) contained in the photocurable composition is from 1% by mass to 30% by mass with respect to a total content of (meth) acrylic monomer components and the compound (C) contained in the photocurable composition.
<12> The photocurable composition according to any one of <1> to <11>, in which a siloxane bond concentration in the photocurable composition is from 0.100 mmol/g to 3.000 mmol/g.
<13> The photocurable composition according to any one of <1> to <12>, in which an aromatic ring concentration in (meth)acrylic monomer components contained in the photocurable composition is from 0.0015 mol/g to 0.0070 mol/g.
<14> The photocurable composition according to any one of <1> to <13>, which is a photocurable composition for stereolithography.
<15> The photocurable composition according to any one of <1> to <14>, which is a photocurable composition used for manufacturing a casting mold by stereolithography.
<16> A three-dimensional object, including a cured article of the photocurable composition according to any one of <1> to <15>.
<17> A casting mold, including the three-dimensional object according to <16>.
<18> The casting mold according to <17>, used for manufacturing a dental prosthesis.
<19> A method for manufacturing a cured article, including a step of polymerizing a curable composition in the casting mold according to <17> or <18>.
<20> A method for manufacturing a dental prosthesis, the method including:

a step of curing the photocurable composition according to any one of <1> to <15> by stereolithography to produce a casting mold used for manufacturing a dental prosthesis; and
a step of polymerizing a curable composition in the casting mold to manufacture the dental prosthesis.

Advantageous Effects of Invention

According to an aspect of the disclosure, there is provided a photocurable composition capable of producing a three-dimensional object in which surface roughness is suppressed, as well as a three-dimensional object using the photocurable composition, a casting mold, a method for manufacturing a cured article, and a method for manufacturing a dental prosthesis, each using the photocurable composition.

BRIEF DESCRIPTION OF DRAWINGS

Fig. 1 is a schematic configuration view of a three-dimensional object A1 formed in Examples.

DESCRIPTION OF EMBODIMENTS

In the disclosure, a numerical range indicated using "to" means a range including numerical values stated before and after "to" as a lower limit value and an upper limit value.
In the disclosure, the amount of each component contained in the composition means, unless otherwise specified, the total amount of the plurality of substances present in the composition, in a case where the plurality of substances corresponding to each component in the composition are present.
In the numerical ranges described step by step in the disclosure, the upper limit value or the lower limit value of one numerical range may be replaced with the upper limit value or the lower limit value of another numerical range described step by step. Moreover, in the numerical ranges described in the disclosure, the upper limit value or the lower limit value of the numerical ranges may be replaced with the values shown in Examples.
In the disclosure, "light" is a concept including active energy rays such as ultraviolet rays and visible rays.
In the disclosure, "(meth)acrylate" means acrylate or methacrylate, "(meth)acryloyl" means acryloyl or methacryloyl, and "(meth)acryl" means acryl or methacryl.

[Photocurable Composition]

A photocurable composition of the disclosure includes: a (meth)acrylic monomer (A) having one or more (meth) acryloyl groups and not having a siloxane bond; a (meth)acrylic monomer (B) having two or more (meth)acryloyl groups and a siloxane bond; a compound (C) not having a (meth)acryloyl group and having a solubility parameter (SP value) of from 7.00 to 15.00 $(cal/cm^3)^{1/2}$; and a photopolymerization initiator.
The photocurable composition of the disclosure includes: the (meth)acrylic monomer components including the (meth)acrylic monomer (A) and the (meth)acrylic monomer (B); the compound (C) satisfying the solubility parameter conditions; and a photopolymerization initiator. As a result, it is possible to produce a three-dimensional object in which surface roughness is suppressed.
The photocurable composition of the disclosure is a composition that cures by light irradiation, and a cured article can be obtained by curing this composition. The manufacturing method when manufacturing a cured article using the photocurable composition of the disclosure is preferably stereolithography.
The photocurable composition of the disclosure is preferably a photocurable composition for stereolithography, in other words, the cured article manufactured using the photocurable composition of the disclosure is preferably a stereolithographic object (that is, a cured article obtained by stereolithography).
Stereolithography is a method in which a cured article (that is, a stereolithographic object) is obtained by repeating the operation of irradiating a photocurable composition with light to form a cured layer, thereby layering the cured layers.
The stereolithography may be inkjet type stereolithography or liquid tank type stereolithography (that is, stereolithography using a liquid tank).
In inkjet type stereolithography, droplets of the photocurable composition are ejected onto a substrate from an inkjet nozzle, and a cured article is obtained by irradiating the droplets adhered to the substrate with light.
In one example of inkjet type stereolithography, for instance, while a head equipped with an inkjet nozzle and a light source is scanned within a plane, the photocurable composition is ejected onto a substrate from the inkjet nozzle, light is irradiated onto the ejected photocurable composition to form a cured layer, and by repeating these operations, the cured layers are sequentially layered to obtain a cured article (that is, a stereolithographic object).
In liquid tank type stereolithography, a part of the photocurable composition contained in a liquid tank (that is, the uncured photocurable composition in a liquid state, and the same will apply hereinafter) is cured by light irradiation to form a cured layer, and by repeating this operation, the cured layers are layered to obtain a cured article (that is, a stereolithographic object). Liquid tank type stereolithography differs from inkjet type stereolithography in that the liquid tank type stereolithography uses a liquid tank.

Examples of the liquid tank type stereolithography include digital light processing (DLP) type stereolithography, and stereolithography (SLA) type stereolithography.
In the DLP type, the photocurable composition in the liquid tank is irradiated with planar light.
In the SLA type, laser light is scanned over the photocurable composition in the liquid tank.
From the viewpoint of more effectively exhibiting the effect of the photocurable composition of the disclosure, the liquid tank type stereolithography is preferably a DLP type stereolithography.
In one example of DLP-type stereolithography, for instance, a 3D printer (for example, "Cara Print 4.0" manufactured by Kulzer, "Max UV" manufactured by Asiga, etc.) including:

a build table that is movable in the vertical direction;
a tray (that is, a liquid tank) which is disposed below the build table (on the gravity direction side, and the same will apply hereinafter), includes a light-transmissive portion, and contains the photocurable composition; and
a light source (for example, an LED light source) disposed below the tray for irradiating planar light to the photocurable composition in the tray through the light-transmissive portion of the tray is used.

In this example, first, a one-layer gap is provided between the build table and the tray, and this gap is filled with the photocurable composition. Next, planar light is irradiated from below to the photocurable composition filled in the gap through the light-transmissive portion of the tray, and by curing the region irradiated with light, a first cured layer is formed. Next, the gap between the build table and the tray is widened by one additional layer, and the generated space is filled with the photocurable composition. Next, light is irradiated onto the photocurable composition filled in the space in the same manner as the first-layer curing, thereby forming a second cured layer. By repeating the above operations, the cured layers are layered to manufacture a three-dimensional object. In this example, the three-dimensional object may be further cured by additionally irradiating light onto the manufactured three-dimensional object.
For DLP type stereolithography, reference may be made, for example, to the descriptions in Japanese Patent No. 5111880 and Japanese Patent No. 5235056.

<Application>

The application of the photocurable composition of the disclosure is not particularly limited, and, for example, it is preferably a photocurable composition used for manufacturing a casting mold, dental products, and the like by stereolithography, and more preferably a photocurable composition used for manufacturing a casting mold by stereolithography.
Examples of the casting mold include a casting mold used for manufacturing a denture with a base. For example, a denture with a base may be manufactured by injecting a curable composition for producing a denture base into the casting mold in a state where artificial teeth are arranged in the casting mold, and curing the curable composition after injection.
Examples of the dental product include a dental prosthesis, a medical instrument used in the oral cavity, a dental model, and a model for lost-mold casting.
Examples of the dental prosthesis include inlays, crowns, bridges, temporary crowns, temporary bridges, and dentures (for example, complete dentures (full dentures), partial dentures (partial denture with a base), and the like).
Examples of the medical instrument used in the oral cavity include dentures (for example, complete dentures (full dentures), partial dentures (partial denture with a base), and the like), mouthpieces, mouthguards, orthodontic appliances, splints such as occlusal splints and splints for temporomandibular disorder treatment, impression trays, and surgical guides.
Examples of the dental models include tooth jaw models.
**In** the photocurable composition of the disclosure, the aromatic ring concentration in (meth)acrylic monomer component is preferably from 0.0015 mol/g to 0.0070 mol/g, more preferably from 0.0016 mol/g to 0.0065 mol/g, and still more preferably from 0.0017 mol/g to 0.0052 mol/g. By adjusting the aromatic ring concentration in (meth)acrylic monomer components as described above, there is a tendency to achieve excellent removability from the casting mold and to suitably suppress deformation of the casting mold during use.
The photocurable composition of the disclosure contains the (meth)acrylic monomer component such as the (meth)acrylic monomer (A) and the (meth)acrylic monomer (B) described later.
**In** the disclosure, the (meth)acrylic monomer component refers to a compound having one or more (meth) acryloyl groups.
From the viewpoint of mechanical strength in the cured article, the content of the (meth)acrylic monomer component with respect to the total amount of the photocurable composition of the disclosure is preferably 60% by mass or more, more preferably 70% by mass or more, and still more preferably 80% by mass or more.
The content of the (meth)acrylic monomer component with respect to the total amount of the photocurable composition of the disclosure may be 99% by mass or less, 95% by mass or less, or 90% by mass or less.

<(Meth)Acrylic Monomer (A)>

The photocurable composition of the disclosure contains at least one (meth)acrylic monomer (A) having one or more (meth)acryloyl groups and not having siloxane bond.

The (meth)acrylic monomer (A) may contain at least one of the following di(meth)acrylic monomer (A-1) and mono(meth)acrylic monomer (A-2), and preferably contains both of the monomers.

Di(meth)acrylic monomer (A-1): A compound having at least one of a ring structure or a urethane bond, and two (meth) acryloyloxy groups, and not having a siloxane bond

Mono(meth)acrylic monomer (A-2): A compound having one (meth)acryloyloxy group and an aromatic ring, and not having a siloxane bond

(Di(Meth)Acrylic Monomer (A-1))

The (meth)acrylic monomer (A) preferably contains the above-described di(meth)acrylic monomer (A-1). The di(meth)acrylic monomer (A-1) having a ring structure suitably contributes to suppression of deformation of a casting mold and improvement of water resistance of the casting mold at the time of manufacturing a denture with a base. When the ring structure contains an aromatic ring, the aromatic ring concentration in (meth)acrylic monomer component tends to increase, releasability tends to increase, and toughness tends to be excellent. The di(meth)acrylic monomer (A-1) having a urethane bond can suppress breakage when a denture with a base is taken out from the casting mold, and tends to be excellent in toughness.

The (meth)acrylic monomer (A) may contain one type of di(meth)acrylic monomer (A-1) or two or more types of di(meth)acrylic monomers (A-1).

The molecular weight or the weight average molecular weight of the di(meth)acrylic monomer (A-1) may be from 400 to 8,000. The weight average molecular weight of the di(meth)acrylic monomer (A-1) may be from 400 to 6,000 or from 400 to 5,000.

In the disclosure, the weight average molecular weight is measured by gel permeation chromatography (GPC).

Two types of di(meth)acrylic monomers (A-1) having different molecular weights may be used in combination. As a result, the dispersibility of the di(meth)acrylic monomer (A-1) in the polymer tends to increase, and the shape recovery rate tends to improve.

The solubility parameter (SP value) of the di(meth)acrylic monomer (A-1) is preferably from 8.50 to 12.70 $(cal/cm^3)^{1/2}$, more preferably from 8.70 to 12.50 $(cal/cm^3)^{1/2}$, and still more preferably from 9.00 to 12.00 $(cal/cm^3)^{1/2}$.

When the solubility parameter (SP value) of the di(meth)acrylic monomer (A-1) is 8.50 $(cal/cm^3)^{1/2}$ or more, a three-dimensional object having less surface roughness and excellent appearance is easily obtained.

When the solubility parameter (SP value) of the di(meth)acrylic monomer (A-1) is 12.70 $(cal/cm^3)^{1/2}$ or less, a three-dimensional object excellent in flexural strength and toughness is easily obtained.

The di(meth)acrylic monomer (A-1) is not particularly limited as long as the di(meth)acrylic monomer (A-1) is a compound having at least one of a ring structure or a urethane bond, and two (meth)acryloyloxy groups, and not having a siloxane bond. Examples of the ring structure include an aromatic ring structure and an alicyclic structure.

The di(meth)acrylic monomer (A-1) may be a compound represented by the following Formula (6-1) when containing a urethane bond, or may be a compound represented by the following Formula (6-2) when containing a ring structure. When the di(meth)acrylic monomer (A-1) contains both a urethane bond and a ring structure, the di(meth)acrylic monomer (A-1) may be a compound represented by the following Formula (6-1).

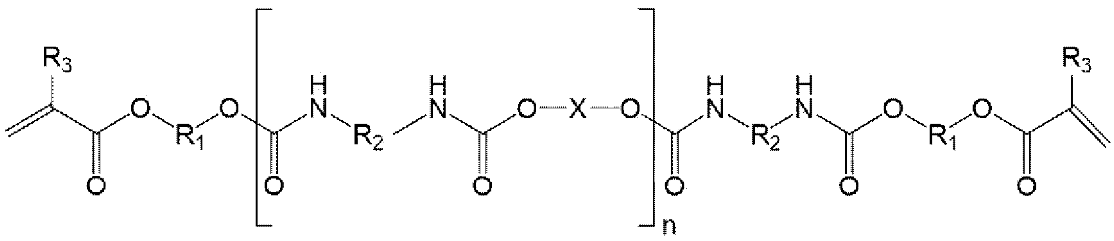 (6-1)

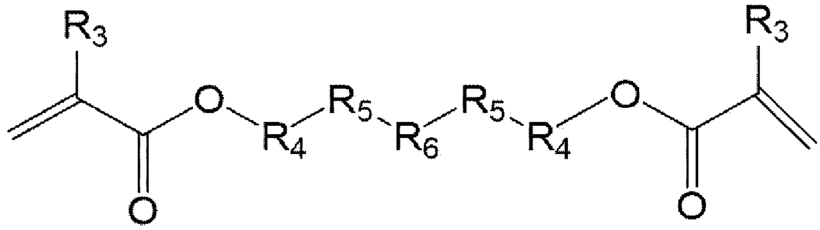
(6-2)

In Formula (6-1), each $R_1$ independently represents a divalent organic group, each $R_2$ independently represents a divalent hydrocarbon group which may have a substituent, each $R_3$ independently represents a hydrogen atom or a methyl group, X represents a divalent organic group, and n represents an integer from 0 to 10. A plurality of $R_1$'s, $R_2$'s, and $R_3$'s may be the same as or different from each other, and when a plurality of X are present, the X's may be the same as or different from each other.

In Formula (6-1), examples of $R_1$ include a divalent hydrocarbon group and a divalent organic group containing one or more bonds selected from the group consisting of an ether bond, and an ester bond.

The divalent hydrocarbon group as $R_1$ may be, for example, a divalent chain hydrocarbon group or a divalent hydrocarbon group containing a ring structure (aromatic ring structure, alicyclic structure).

When the divalent organic group as $R_1$ contains an ether bond, the number of ether bonds is preferably from 1 to 30, more preferably from 1 to 20, and still more preferably from 1 to 15.

When the divalent organic group as $R_1$ contains an ester bond, the number of ester bonds is preferably from 1 to 12, more preferably from 1 to 8, and still more preferably from 1 to 6.

In Formula (6-1), the number of carbon atoms of $R_1$ may be, for example, from 2 to 60, but is preferably from 2 to 40, and more preferably from 2 to 30.

In Formula (6-1), $R_1$ is preferably a group represented by the following General Formula (6-1-$R_1$).

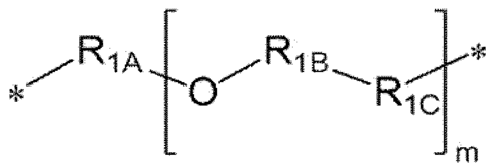
(6-1-$R_1$)

In General Formula (6-1-$R_1$), $R_{1A}$ is a divalent hydrocarbon group which may have a substituent, $R_{1B}$ is a methylene group ($-CH_2-$) or a carbonyl group (-C(=O)-), $R_{1C}$ is a divalent hydrocarbon group, and m is an integer of from 0 to 10. When a plurality of $R_{1B}$ and $R_{1C}$ are present, the plurality of $R_{1B}$ and $R_{1C}$ may be the same as or different from each other. * indicates a bonding position.

The divalent hydrocarbon group which may have a substituent as $R_{1A}$ in General Formula (6-1-$R_1$) is, for example, a divalent chain hydrocarbon group or a divalent hydrocarbon group containing a ring structure, and is preferably a divalent chain hydrocarbon group. The ring structure contained in the divalent hydrocarbon group may be an aromatic structure or an alicyclic structure.

The divalent chain hydrocarbon group as $R_{1A}$ may be branched, saturated, or unsaturated.

The number of carbon atoms of the divalent chain hydrocarbon group as $R_{1A}$ is preferably from 2 to 20, more preferably from 2 to 15, and still more preferably from 2 to 10.

Substituents which the divalent chain hydrocarbon group as $R_{1A}$ may have are, for example, an alkoxy group having from 2 to 10 carbon atoms and an aryloxy group having from 6 to 20 carbon atoms, and an aryloxy group is preferable. Examples of the aryloxy group include a phenoxy group and a naphthyloxy group, and a phenoxy group is preferable.

The divalent hydrocarbon group containing an aromatic structure as $R_{1A}$ is preferably a divalent hydrocarbon group having an aromatic structure having from 6 to 25 carbon atoms (the number of carbon atoms is more preferably from 6 to 20, and still more preferably from 6 to 15). Examples of the substituent that the divalent hydrocarbon group containing an aromatic structure may have include a linear or branched alkyl group having from 1 to 6 carbon atoms.

The divalent hydrocarbon group containing an alicyclic structure as $R_{1A}$ is preferably a divalent hydrocarbon group having an alicyclic structure having from 3 to 20 (the number of carbon atoms is more preferably from 6 to 12, and still more preferably from 6 to 8) carbon atoms. Examples of the substituent that the divalent hydrocarbon group containing an alicyclic structure may have include a linear or branched alkyl group having from 1 to 6 carbon atoms.

Examples of the divalent hydrocarbon group as $R_{1C}$ in General Formula (6-1-$R_1$) include a divalent chain hydrocarbon group and a divalent hydrocarbon group containing a ring structure (aromatic structure or alicyclic structure), and a divalent chain hydrocarbon group is preferable.

The number of carbon atoms of the divalent chain hydrocarbon group as $R_{1C}$ is preferably from 1 to 30, more preferably from 1 to 20, and still more preferably from 1 to 10.

The divalent hydrocarbon group containing an aromatic structure as $R_{1C}$ is preferably a divalent hydrocarbon group having an aromatic structure having from 6 to 25 carbon atoms (the number of carbon atoms is more preferably from 6 to 20, and still more preferably from 6 to 15).

The divalent hydrocarbon group containing an alicyclic structure as $R_{1C}$ is preferably a divalent hydrocarbon group having an alicyclic structure having from 3 to 20 (the number of carbon atoms is more preferably from 6 to 12, and still more preferably from 6 to 8) carbon atoms.

m in General Formula (6-1-$R_1$) is an integer from 0 to 10, preferably from 0 to 8, and more preferably from 0 to 6.

The left symbol * (that is, the bonding position from $R_{1A}$) in General Formula (6-1-$R_1$) is preferably bonded to the oxygen atom on the left side of $R_1$ in General Formula (6-1), and the right symbol * (that is, the bonding position from $R_{1C}$) is preferably bonded to the oxygen atom on the right side of $R_1$ in General Formula (6-1).

In Formula (6-1), the divalent hydrocarbon group as $R_2$ is, for example, a divalent chain hydrocarbon group or a divalent hydrocarbon group containing a ring structure.

The number of carbon atoms of the divalent chain hydrocarbon group as $R_2$ is preferably from 1 to 25, more preferably from 1 to 20, and still more preferably from 2 to 15.

The divalent hydrocarbon group containing a ring structure as $R_2$ may contain an aromatic structure and may contain an alicyclic structure.

The number of carbon atoms of the divalent hydrocarbon group containing an aromatic structure as $R_2$ is preferably from 6 to 25, more preferably from 6 to 20, and still more preferably from 6 to 15.

The number of carbon atoms of the divalent hydrocarbon group containing an alicyclic structure as $R_2$ is preferably from 3 to 20, more preferably from 6 to 15, and still more preferably from 6 to 10.

Specifically, the divalent hydrocarbon group as $R_2$ is preferably a divalent hydrocarbon group represented by a structure selected from the following General Formulas (6-1-$R_2$-1) to (6-1-$R_2$-13). From the viewpoint of obtaining a three-dimensional object excellent in flexibility and toughness, a divalent hydrocarbon group represented by a structure selected from the following General Formulas (6-1-$R_2$-4) to (6-1-$R_2$-13) is more preferable, and a divalent hydrocarbon group represented by a structure selected from the following General Formulas (6-1-$R_2$-4) to (6-1-$R_2$-7) is still more preferable.

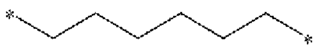
(6-1-$R_2$-1)

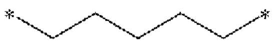
(6-1-$R_2$-2)

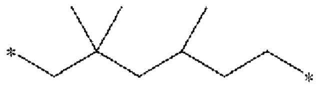
(6-1-$R_2$-3)

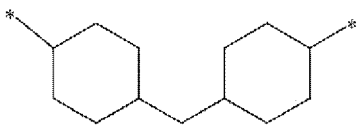
(6-1-$R_2$-4)

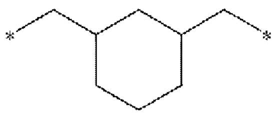
(6-1-$R_2$-5)

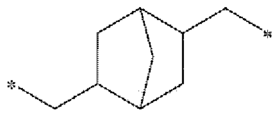
(6-1-$R_2$-6)

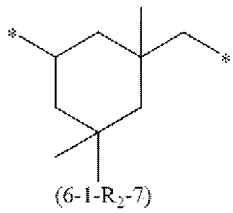
(6-1-$R_2$-7)

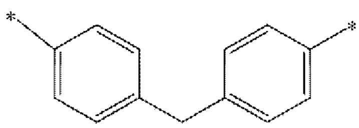
(6-1-$R_2$-8)

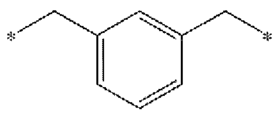
(6-1-$R_2$-9)

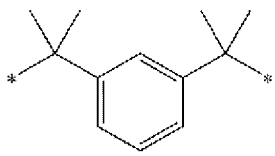
(6-1-$R_2$-10)

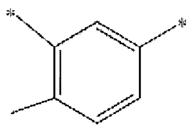
(6-1-$R_2$-11)

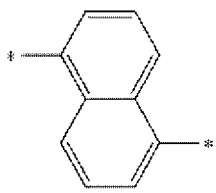
(6-1-$R_2$-12)

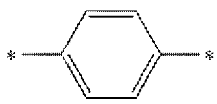
(6-1-$R_2$-13)

In General Formulas (6-1-$R_2$-1) to (6-1-$R_2$-13), * indicates a bonding position.

In Formula (6-1), X is a divalent organic group, and preferably a divalent organic group containing one or more bonds selected from the group consisting of an ether bond and an ester bond.

When the divalent organic group as X contains an ether bond, the number of ether bonds is preferably from 1 to 100, more preferably from 2 to 90, and still more preferably from 4 to 80.

When the divalent organic group as X contains an ester bond, the number of ester bonds is preferably from 1 to 100, more preferably from 2 to 90, and still more preferably from 4 to 80.

The number of carbon atoms of the divalent organic group as X is preferably from 2 to 500, more preferably from 2 to 400, and still more preferably from 2 to 300.

X in Formula (6-1) is preferably a group represented by the following General Formula (6-1-X).

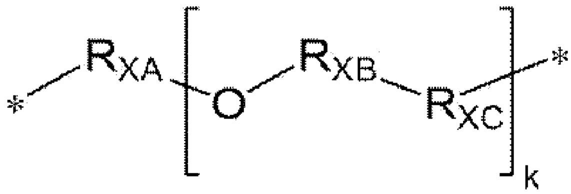

(6-1-X)

In General Formula (6-1-X), $R_{XA}$ is a divalent hydrocarbon group, $R_{XB}$ is a methylene group ($-CH_2-$) or a carbonyl group (-C(=O)-), $R_{XC}$ is a divalent hydrocarbon group, and k is an integer from 0 to 100. When a plurality of $R_{XB}$ and $R_{XC}$ are present, the plurality of $R_{XB}$ and $R_{XC}$ may be the same as or different from each other. * indicates a bonding position.

The divalent hydrocarbon group as $R_{XA}$ in General Formula (6-1-X) is, for example, a divalent chain hydrocarbon group or a divalent hydrocarbon group containing a ring structure, and is preferably a divalent chain hydrocarbon group. The ring structure contained in the divalent hydrocarbon group may be an aromatic structure or an alicyclic structure.

The divalent chain hydrocarbon group as $R_{XA}$ may be branched, saturated, or unsaturated.

The number of carbon atoms of the divalent chain hydrocarbon group as $R_{XA}$ is preferably from 2 to 20, more preferably from 2 to 15, and still more preferably from 2 to 10.

The divalent hydrocarbon group containing an aromatic structure as $R_{XA}$ is preferably a divalent hydrocarbon group having an aromatic structure having from 6 to 25 carbon atoms (the number of carbon atoms is more preferably from 6 to 20, and still more preferably from 6 to 15).

The divalent hydrocarbon group containing an alicyclic structure as $R_{XA}$ is preferably a divalent hydrocarbon group having an alicyclic structure having from 3 to 20 (the number of carbon atoms is more preferably from 6 to 12, and still more preferably from 6 to 8) carbon atoms.

Examples of the divalent hydrocarbon group as $R_{XC}$ in General Formula (6-1-X) include a divalent chain hydrocarbon group and a divalent hydrocarbon group containing a ring structure, and a divalent chain hydrocarbon group is preferable.

The number of carbon atoms of the divalent chain hydrocarbon group as $R_{XC}$ is preferably from 1 to 30, more preferably from 1 to 20, and still more preferably from 1 to 10.

The divalent hydrocarbon group containing an aromatic structure as $R_{XC}$ is preferably a divalent hydrocarbon group having an aromatic structure having from 6 to 25 carbon atoms (the number of carbon atoms is more preferably from 6 to 20, and still more preferably from 6 to 15).

The divalent hydrocarbon group containing an alicyclic structure as $R_{XC}$ is preferably a divalent hydrocarbon group having an alicyclic structure having from 3 to 20 (the number of carbon atoms is more preferably from 6 to 12, and still more preferably from 6 to 8) carbon atoms.

k in General Formula (6-1-X) is from 0 to 100, preferably from 2 to 90, and more preferably from 4 to 80.

In Formula (6-2), each $R_3$ independently represents a hydrogen atom or a methyl group, each $R_4$ independently represents an alkylene group which may have a substituent, an alkyleneoxy group, or a combination thereof, each $R_5$ independently represents an oxygen atom or an ester bond (*1-O-C(=O)-*2, *1 is a bonding position with $R_4$, and *2 is a bonding position with $R_6$), and $R_6$ represents a divalent linking group having a ring structure.

In Formula (6-2), $R_4$ may be an alkyleneoxy group and $R_5$ may be an oxygen atom, or $R_4$ may be an alkylene group and $R_5$ may be an ester bond (*1-O-C(=O)-*2, *1 is a bonding position with $R_4$, and *2 is a bonding position with $R_6$). $R_6$ may be a phenylene group or a bisphenol skeleton (for example, a bisphenol A skeleton or a bisphenol F skeleton).

The content of the di(meth)acrylic monomer (A-1) contained in the photocurable composition is preferably from 3% by mass to 50% by mass, more preferably from 5% by mass to 45% by mass, and still more preferably from 10% by mass to 40% by mass with respect to the total content of (meth)acrylic monomer components and the compound (C) contained in the photocurable composition.

(Mono(Meth)Acrylic Monomer (A-2))

The (meth)acrylic monomer (A) preferably contains the above-described mono(meth)acrylic monomer (A-2). When the mono(meth)acrylic monomer (A-2) is used, the aromatic ring concentration in (meth)acrylic monomer component increases, the denture with a base tends to be easily taken out from the casting mold (releasability tends to increase), and breakage when taking out the denture with a base from the casting mold tends to be suppressed (tends to be excellent in toughness). Furthermore, it is possible to suitably produce a casting mold in which deformation at the time of manufacturing a denture with a base is suppressed, it is possible to improve the flexibility of the casting mold by using a monofunctional monomer, and thus it is also possible to improve the shape recoverability of the casting mold.

The (meth)acrylic monomer (A) may contain one type of mono(meth)acrylic monomer (A-2) or two or more types of mono(meth)acrylic monomers (A-2).

The molecular weight or the weight average molecular weight of the mono(meth)acrylic monomer (A-2) may be from 160 to 400 or may be 180 to 300.

The solubility parameter (SP value) of the mono(meth)acrylic monomer (A-2) is preferably from 8.80 to 13.00 $(cal/cm^3)^{1/2}$, more preferably from 9.00 to 12.50 $(cal/cm^3)^{1/2}$, and still more preferably from 9.50 to 12.00 $(cal/cm^3)^{1/2}$.

When the solubility parameter (SP value) of the mono(meth)acrylic monomer (A-2) is 8.80 $(cal/cm^3)^{1/2}$ or more, a three-dimensional object having less surface roughness and excellent appearance is easily obtained.

When the solubility parameter (SP value) of the mono(meth)acrylic monomer (A-2) is 13.00 $(cal/cm^3)^{1/2}$ or less, a three-dimensional object excellent in flexural strength and toughness is easily obtained.

The mono(meth)acrylic monomer (A-2) is not particularly limited as long as the mono(meth)acrylic monomer (A-2) is a compound having one (meth)acryloyloxy group and an aromatic ring, and may be, for example, a compound represented by the following Formula (5).

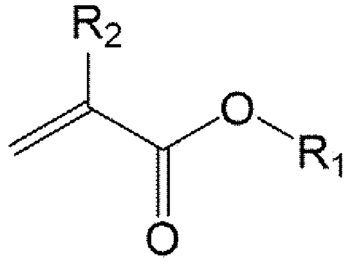

(5)

**In** Formula (5), $R_1$ is a monovalent organic group having an aromatic ring, and $R_2$ is a hydrogen atom or a methyl group.

$R_1$ in Formula (5) preferably has from 6 to 30 carbon atoms, and more preferably has from 6 to 20 carbon atoms.

The monovalent organic group as $R_1$ in Formula (5) may have one or more aromatic rings, but preferably has one or two aromatic rings.

The aromatic ring of the monovalent organic group as $R_1$ in Formula (5) may be a single ring or multiple rings.

Examples of the monocyclic aromatic ring include a phenyl structure (phenyl group, phenylene group). The monocyclic aromatic ring may have a substituent, and examples of the substituent include an alkyl group, an aryloxy group (phenoxy group or the like), an arylalkylene group, and an aryl group.

Examples of the polycyclic aromatic ring include a naphthyl structure (naphthyl group, naphthylene group). The polycyclic aromatic ring may have a substituent, and examples of the substituent include an alkyl group, an aryloxy group (phenoxy group or the like), an arylalkylene group, and an aryl group.

$R_1$ is preferably a monovalent organic group represented by a structure selected from the following General Formulas (5-$R_1$-1) to (5-$R_1$-7).

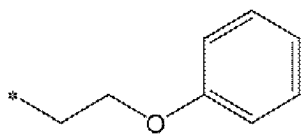

(5-$R_1$-1)

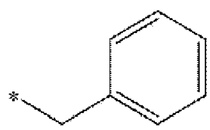

(5-$R_1$-2)

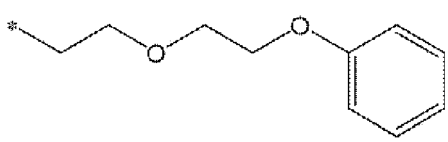

(5-$R_1$-3)

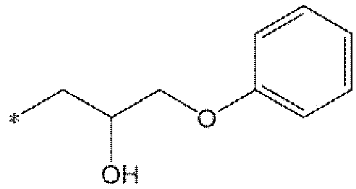

(5-$R_1$-4)

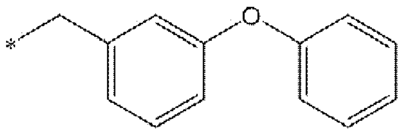

(5-$R_1$-5)

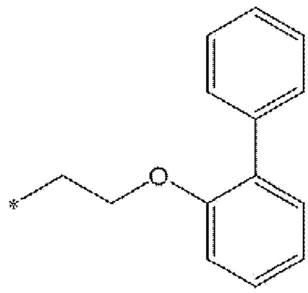

(5-$R_1$-6)

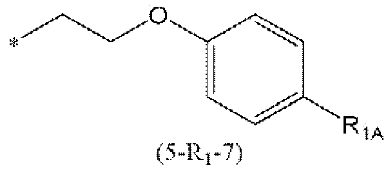

(5-$R_1$-7)

In General Formulas (5-$R_1$-1) to (5-$R_1$-7), * indicates a bonding position.

In General Formula (5-$R_1$-7), $R_{1A}$ represents an alkyl group having from 1 to 10 carbon atoms.

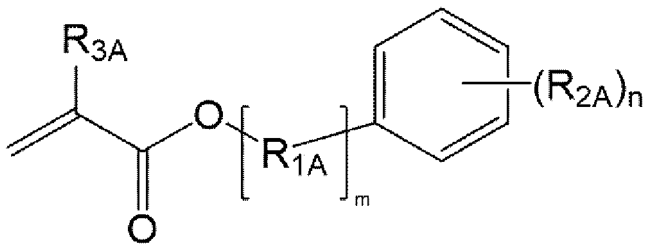

(5-1)

In Formula (5-1), $R_{1A}$ is a divalent linking group, $R_{2A}$ is an alkyl group or an aryl group which may have a substituent, $R_{3A}$ is a hydrogen atom or a methyl group, n is an integer from 0 to 5, and m is 0 or 1.

In Formula (5-1), $R_{1A}$ may be an alkylene group, an arylene group, an alkylarylene group, an arylenealkyl group, an alkyleneoxy group, an aryleneoxy group, or a combination of two or more thereof.

The number of carbon atoms of $R_{1A}$ is preferably from 1 to 20, and more preferably from 1 to 10.

The hydrogen atom contained in $R_{1A}$ may be substituted with a hydroxy group, an alkyl group, an aryl group, an amino group, or the like.

**In** Formula (5-1), n is preferably 0 or 1. When n is from 1 to 5, $R_2$ is preferably a phenyl group which may have a substituent.

The content of the mono(meth)acrylic monomer (A-2) contained in the photocurable composition is preferably from 20% by mass to 85% by mass, more preferably from 30% by mass to 80% by mass, and still more preferably from 40% by mass to 70% by mass with respect to the total content of (meth)acrylic monomer components and the compound (C) contained in the photocurable composition.

<(Meth)Acrylic Monomer (B)>

The photocurable composition of the disclosure contains at least one (meth)acrylic monomer (B) having two or more (meth)acryloyl groups and a siloxane bond. When the (meth)acrylic monomer (B) is contained, the aromatic ring

concentration in (meth)acrylic monomer component tends to increase, the denture with a base tends to be easily taken out from the casting mold (releasability tends to increase), and the denture with a base tends to be excellent in deformability and dimensional accuracy at the time of manufacturing the denture with a base.

The (meth)acrylic monomer (B) has a siloxane bond (Si-O-Si) and two or more (meth)acryloyloxy groups. The (meth)acrylic monomer (B) may contain a plurality of siloxane bonds (Si-O-Si), and more specifically, may contain a linear siloxane bond, a ladder-like siloxane bond including a linear chain and a branch, a cage-type siloxane bond, or the like. The (meth)acrylic monomer (B) may contain two or three or more (meth)acryloyloxy groups.

Examples of the siloxane bond include a dimethylsiloxane bond, a methylphenylsiloxane bond, and a diphenylsiloxane bond, and a dimethylsiloxane bond is preferable.

The (meth)acrylic monomer (B) may be a compound containing a siloxane bond (Si-O-Si) and three or more (meth)acryloyloxy groups, or may be a silsesquioxane containing three or more (meth)acryloyloxy groups.

The molecular weight of the (meth)acrylic monomer (B) may be from 400 to 5,000.

The weight average molecular weight of the (meth)acrylic monomer (B) may be from 400 to 4,000.

By combining the (meth)acrylic monomer (B) and the compound (C) described later, it becomes easy to produce a three-dimensional object in which the surface roughness is suppressed. As a result, a three-dimensional object having excellent releasability such as ease of mold release and toughness at the time of mold release, less surface roughness, and excellent appearance is easily obtained.

The solubility parameter (SP value) of the (meth)acrylic monomer (B) is preferably from 6.50 to 9.50 $(cal/cm^3)^{1/2}$, and more preferably from 7.50 to 9.50 $(cal/cm^3)^{1/2}$.

When the solubility parameter (SP value) of the (meth)acrylic monomer (B) is 6.50 $(cal/cm^3)^{1/2}$ or more, a three-dimensional object having less surface roughness and excellent appearance is easily obtained.

When the solubility parameter (SP value) of the (meth)acrylic monomer (B) is 9.50 $(cal/cm^3)^{1/2}$ or less, a three-dimensional object having excellent releasability is easily obtained.

The (meth)acrylic monomer (B) may be a compound represented by the following Formula (7).

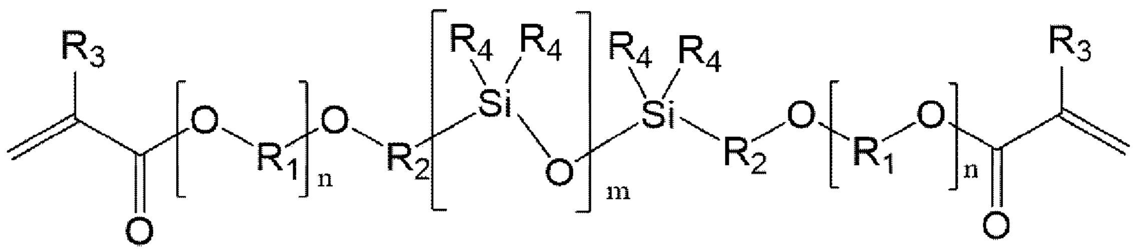

(7)

In Formula (7), each $R_1$ independently represents an alkylene group which may have a substituent, each $R_2$ independently represents an alkylene group which may have a substituent, each $R_3$ independently represents a hydrogen atom or a methyl group, each $R_4$ independently represents an alkyl group, a hydrogen atom or an aryl group, m is an integer of 0 or more, and n is an integer of 0 or more.

$R_1$ is preferably a methylene group, an ethylene group, or a propylene group, and $R_2$ is more preferably a methylene group, an ethylene group, a propylene group, or a butylene group.

$R_4$ is preferably a methyl group or a phenyl group, and more preferably a methyl group.

m may be from 1 to 30 or from 2 to 20. From the viewpoint of compatibility with other (meth)acrylic monomer components, m is preferably 30 or less, and more preferably 20 or less.

n may be 0 or 1 or more.

The content of the (meth)acrylic monomer (B) contained in the photocurable composition is preferably from 1% by mass to 35% by mass, more preferably from 5% by mass to 30% by mass, and still more preferably from 10% by mass to 25% by mass, with respect to the total content of (meth)acrylic monomer components and the compound (C) contained in the photocurable composition.

**In** (meth)acrylic monomer components, the total content of the (meth)acrylic monomer (A) and the (meth)acrylic monomer (B) is preferably from 50% by mass to 100% by mass, more preferably from 70% by mass to 100% by mass, and still more preferably from 90% by mass to 100% by mass.

The siloxane bond concentration in the photocurable composition is preferably from 0.100 mmol/g to 3.000 mmol/g, more preferably from 0.300 mmol/g to 2.500 mmol/g, and still more preferably from 0.400 mmol/g to 2.000 mmol/g.

When the siloxane bond concentration is 0.100 mmol/g or more, releasability tends to be improved, and when the siloxane bond concentration is 3.000 mmol/g or less, breakage when a dental prosthesis such as a denture with a base is taken out from a casting mold can be suppressed, and toughness tends to be excellent.

The photocurable composition of the disclosure may or may not contain a photopolymerizable component other

than (meth)acrylic monomer components.

Examples of the photopolymerizable component other than (meth)acrylic monomer components include styrene, styrene derivatives, and (meth)acrylonitrile.

**In** the photocurable composition of the disclosure, the content of the photopolymerizable component other than (meth)acrylic monomer components may be 20% by mass or less, 10% by mass or less, 5% by mass or less, or 1.0% by mass or less, with respect to the total amount of the photopolymerizable components in the photocurable composition of the disclosure.

The lower limit of the content of the photopolymerizable component other than (meth)acrylic monomer components is not particularly limited, and may be, for example, 0% by mass or more.

The solubility parameters (SP values) of (meth)acrylic monomer components contained in the photocurable composition or the solubility parameters (SP values) of the photopolymerizable components contained in the photocurable composition may independently be in the range of from 8.00 to 12.00 $(cal/cm^3)^{1/2}$, from 8.50 to 11.50 $(cal/cm^3)^{1/2}$, or from 9.00 to 11.00 $(cal/cm^3)^{1/2}$.

The solubility parameter (SP value) of (meth)acrylic monomer components and the solubility parameter (SP value) of the photopolymerizable components each mean a weighted average value.

The standard deviation of the solubility parameters (SP values) of (meth)acrylic **monomer** components or the standard deviation of the solubility parameters (SP values) of the photopolymerizable components is each independently preferably 1.3 or less, more preferably 1.1 or less, and still more preferably less than 1.0.

<Compound (C)>

The photocurable composition of the disclosure does not have a (meth)acryloyl group and includes a compound (C) having a solubility parameter (SP value) of from 7.00 to 15.00 $(cal/cm^3)^{1/2}$.

The solubility parameter (SP value) of the compound (C) is from 7.00 to 15.00 $(cal/cm^3)^{1/2}$, and is preferably from 8.00 to 13.00 $(cal/cm^3)^{1/2}$, more preferably from 8.30 to 11.00 $(cal/cm^3)^{1/2}$, and still more preferably from 8.30 to 10.70 $(cal/cm^3)^{1/2}$ from the viewpoint of suppressing the surface roughness of the three-dimensional object and ease of mold release.

When the photocurable composition contains two or more types of compounds (C), the solubility parameter (SP value) of the compound (C) means a weighted average value of the solubility parameters of the two or more types of compounds (C).

The compound (C) is preferably not a compound that is excited by light in a specific wavelength range (for example, from 365 nm to 500 nm) to generate a radical, and is preferably a compound having no terminal unsaturated bond.

The compound (C) is not particularly limited as long as the compound (C) is a compound having a solubility parameter (SP value) satisfying from 7.00 to 15.00 $(cal/cm^3)^{1/2}$, and examples thereof include alcohol compounds, carboxylic acids, ester compounds, and polyether compounds.

Examples of the alcohol compound include higher alcohols, monoalcohol esters, polyhydric alcohols, and polyhydric alcohol esters.

As the higher alcohol, an alcohol compound having 5 or more carbon atoms is preferable, an alcohol compound having 6 or more carbon atoms is more preferable, and an alcohol compound having from 6 to 12 carbon atoms is still more preferable. The higher alcohol may be an alcohol compound having one hydroxy group, or may be a linear alcohol compound having one hydroxy group.

Specific examples of the higher alcohol include 1-pentanol, 1-hexanol, 1-heptanol, **1-**octanol, 1-nonanol, 1-decanol, 1-undecanol, and 1-dodecanol (lauryl alcohol).

Examples of the monoalcohol ester include a monoester containing one hydroxy group, a diester containing one hydroxy group, and a triester containing one hydroxy group.

Specific examples of the monoalcohol ester include oxyacid esters such as diisostearyl malate and triethyl citrate.

Examples of the polyhydric alcohol include compounds containing two or more hydroxy groups and having no ester bond. The number of hydroxy groups of the polyhydric alcohol may be two or more, and may be two or three.

Specific examples of the polyhydric alcohol include trimethylolpropane and the like.

Examples of the polyhydric alcohol ester include compounds containing two or more hydroxy groups and having an ester bond. The number of hydroxy groups of the polyhydric alcohol ester may be two or more, and may be two or three.

Specific examples of the polyhydric alcohol ester include glycerin monoesters such as monoglycerol stearate.

The carboxylic acid is preferably a higher fatty acid having 5 or more carbon atoms, preferably 6 or more carbon atoms. The carboxylic acid may be a higher fatty acid having 20 or less carbon atoms or a higher fatty acid having 18 or less carbon atoms.

Specific examples of the carboxylic acid include lauric acid, myristic acid, palmitic acid, and stearic acid.

Examples of the ester compound include monoesters, diesters, and triesters of higher fatty acids having 5 or more carbon atoms. The ester compound may be a fatty acid ester (fatty acid monoester, fatty acid diester, fatty acid triester, and the like) synthesized from a higher fatty acid and a higher alcohol.

Specific examples of the ester compound include ethyl laurate, isopropyl myristate, isopropyl palmitate, and glyceryl trioleate.

Since a fatty acid ester synthesized from a higher fatty acid and a higher alcohol is easily dimerized, the fatty acid ester may be a dimer in the raw material stage, or may be a derivative of a dimer diol such as dimer dilinoleic acid di(isostearyl/phytosteryl).

Examples of the polyether compound include compounds containing two or more ether bonds.

Specific examples of the polyether compound include polyalkylene glycols such as polyethylene glycol and polypropylene glycol (the alkylene group preferably has from 2 to 4 carbon atoms).

The absolute value of the difference between the average value of the solubility parameters (SP values) of (meth)acrylic monomer components contained in the photocurable composition and the average value of the solubility parameter (SP value) of the compound (C) contained in the photocurable composition is preferably 4.00 $(cal/cm^3)^{1/2}$ or less, more preferably 3.00 $(cal/cm^3)^{1/2}$ or less, and still more preferably 2.00 $(cal/cm^3)^{1/2}$ or less from the viewpoint of suppressing the surface roughness of the three-dimensional object and ease of mold release.

The lower limit of the absolute value of the difference is not particularly limited as long as the lower limit is 0 $(cal/cm^3)^{1/2}$ or more, and may be, for example, 0.10 $(cal/cm^3)^{1/2}$ or more.

In the disclosure, the average value of the solubility parameter (SP value) means a weighted average value of the SP values of the corresponding components.

The difference (SP value 1 - SP value 2) between the average value of the solubility parameters (SP value 1) of (meth)acrylic monomer components contained in the photocurable composition and the average value of the solubility parameter (SP value 2) of the compound (C) contained in the photocurable composition may be -0.50 $(cal/cm^3)^{1/2}$ or more, may be 0 $(cal/cm^3)^{1/2}$ or more, or may be 0.30 $(cal/cm^3)^{1/2}$ or more from the viewpoint of suppressing the surface roughness of the three-dimensional object and ease of mold release.

The upper limit of "SP value 1 - SP value 2" is not particularly limited, and may be, for example, 3.00 $(cal/cm^3)^{1/2}$ or less, or 2.00 $(cal/cm^3)^{1/2}$ or less.

When the photocurable composition contains two or more types of the compound (C), the standard deviation of the solubility parameter (SP value) of the compound (C) is preferably 3.5 or less, more preferably 2.0 or less, and still more preferably less than 1.0.

The average value of the solubility parameters (SP values) of the components (here, photopolymerization initiators are excluded) contained in the photocurable composition is preferably from 8.00 to 12.00 $(cal/cm^3)^{1/2}$, more preferably from 9.00 to 11.00 $(cal/cm^3)^{1/2}$, and still more preferably from 9.00 to 10.30 $(cal/cm^3)^{1/2}$.

The standard deviation of the solubility parameters (SP value) of the components (here, photopolymerization initiators are excluded) contained in the photocurable composition is preferably 1.3 or less, more preferably 1.1 or less, and still more preferably less than 1.0.

The cohesive energy density (cal/mol) of the compound (C) is preferably 10,000 or more, and more preferably 11,000 or more from the viewpoint that the compound (C) is less likely to volatilize and the surface roughness of the three-dimensional object is suppressed.

The cohesive energy density (cal/mol) of the compound (C) is preferably 150,000 or less, and more preferably 110,000 or less from the viewpoint of compatibility with the (meth)acrylic monomer component.

The content of the compound (C) contained in the photocurable composition is preferably from 1% by mass to 30% by mass, more preferably from 3% by mass to 20% by mass, and still more preferably from 5% by mass to 15% by mass, with respect to the total content of (meth)acrylic monomer components and the compound (C) contained in the photocurable composition.

[Photopolymerization Initiator]

The photocurable composition of the disclosure contains a photopolymerization initiator.

The photocurable composition of the disclosure may contain only one type or two or more types of photopolymerization initiators.

The photopolymerization initiator is not particularly limited as long as the photopolymerization initiator generates radicals by irradiating light, but the photopolymerization initiator preferably generates radicals at the wavelength of light used in stereolithography.

The wavelength of light used in stereolithography is generally from 365 nm to 500 nm, but practically preferably from 365 nm to 430 nm, and more preferably from 365 nm to 420 nm.

The photopolymerization initiator is preferably a compound that is not classified as (meth)acrylic monomer components. For example, the photopolymerization initiator is preferably a compound not having (meth)acryloyl group.

The photopolymerization initiator may be a compound that satisfies a solubility parameter (SP value) of from 7.00 to 15.00 $(cal/cm^3)^{1/2}$ or may be a compound that does not satisfy the solubility parameter. In the disclosure, a compound that is excited by light in a specific wavelength range (for example, from 365 nm to 500 nm) and generates radicals is classified as a photopolymerization initiator. A compound that is excited by light in a specific wavelength range (for example, 365 nm to 500 nm), generates radicals, and satisfies a solubility parameter (SP value) of from 7.00 to 15.00 $(cal/cm^3)^{1/2}$ is classified as a photopolymerization initiator, not as the compound (C). That is, in the disclosure, the compound (C) is not a compound that is excited by light in a specific wavelength range (for example, 365 nm to 500 nm) to generate radicals.

Examples of photopolymerization initiators include acylphosphine oxide compounds, alkylated benzoylformate compounds, alkylphenone compounds, titanocene compounds, oxime ester compounds, benzoin compounds, acetophenone compounds, benzophenone compounds, thioxanthone compounds, α-acyloxime ester compounds, phenylglyoxylate compounds, benzyl compounds, azo compounds, diphenyl sulfide compounds, organic dye compounds, iron-phthalocyanine compounds, benzoin ether compounds, and anthraquinone compounds.

Examples of acylphosphine oxide compounds include 2,4,6-trimethylbenzoyl diphenylphosphine oxide, 2,6-dimethoxybenzoyl diphenylphosphine oxide, and bis(2,4,6-trimethylbenzoyl)phenylphosphine oxide.

The total content of the photopolymerization initiator in the photocurable composition of the disclosure is preferably 0.1% by mass to 5% by mass, more preferably 0.5% by mass to 4% by mass, and still more preferably 0.5% by mass to 3% by mass with respect to the total amount of the photocurable composition.

When the photopolymerization initiator contains an acylphosphine oxide compound, the content of the acylphosphine oxide compound may be 50% by mass to 100% by mass, 70% by mass to 100% by mass, or 90% by mass to 100% by mass with respect to the total amount of the photopolymerization initiator.

The total content of (meth)acrylic monomer components, the compound (C), and the photopolymerization initiator, or the total content of the (meth)acrylic monomer (A), the (meth)acrylic monomer (B), the compound (C), and the photopolymerization initiator is each independently preferably 80% by mass or more, more preferably 90% by mass or more, and still more preferably 95% by mass or more with respect to the total amount of the photocurable composition of the disclosure.

The upper limit of the total content of (meth)acrylic monomer components, the compound (C), and the photopolymerization initiator or the total content of the (meth)acrylic monomer (A), the (meth)acrylic monomer (B), the compound (C), and the photopolymerization initiator is not particularly limited, and may be 100% by mass or less.

<Other Components>

The photocurable composition of the disclosure may contain one or more components other than the above-described components as necessary.

Examples of other components include coloring materials, coupling agents such as silane coupling agents, additives such as rubber agents, ion trapping agents, ion exchangers, leveling agents, plasticizers, and antifoaming agents, and thermal polymerization initiators.

When the photocurable composition of the disclosure contains a thermal polymerization initiator, both photocuring and thermal curing can be performed. Examples of the thermal polymerization initiator include a thermal radical generator and an amine compound.

Examples of other components include inorganic fillers.

However, from the viewpoint of further improving the shaping accuracy of the cured article, it is preferable that the photocurable composition of the disclosure either does not contain an inorganic filler (for example, silica, barium borosilicate glass, and the like, and the same will apply hereinafter), or, when the photocurable composition contains an inorganic filler, the content of the inorganic filler with respect to the total amount of the photocurable composition is 60% by mass or less (more preferably 40% by mass or less, still more preferably 20% by mass or less, and particularly preferably 10% by mass or less).

The method for preparing the photocurable composition of the disclosure is not particularly limited.

Examples of the method for preparing the photocurable composition of the disclosure include a method of mixing a (meth)acrylic monomer component, a photopolymerization initiator, and other components as necessary.

Means for mixing the respective components is not particularly limited, and examples thereof include means such as dissolution by ultrasonic waves, a double arm stirrer, a roll kneader, a twin screw extruder, a ball mill kneader, and a planetary stirrer.

The photocurable composition of the embodiment may be prepared by mixing the respective components, filtering the mixture with a filter to remove impurities, and further subjecting the mixture to a vacuum defoaming treatment.

<Preferable Viscosity of Photocurable Composition>

The photocurable composition of the disclosure preferably has a viscosity (hereinafter also simply referred to as "viscosity"), which is measured under the conditions of 25°C and 50rpm by an E-type viscometer, of from 5 mPa·s to 6,000 mPa·s.
Here, rpm means revolutions per minute.
When the viscosity is 5 mPa·s to 6,000 mPa·s, handleability of the photocurable composition when manufacturing a cured article (in particular, a stereolithographic object) is excellent.
The viscosity is more preferably 10 mPa·s to 5,000 mPa·s, still more preferably 20 mPa·s to 5,000 mPa·s, and still more preferably 100 mPa·s to 4,500 mPa·s.

[Three-dimensional object]

The three-dimensional object of the disclosure includes a cured article of the photocurable composition of the disclosure.
Therefore, in a case where the three-dimensional object of the disclosure is a casting mold, it is possible to suppress deformation at the time of manufacturing a dental prosthesis such as a denture with a base, and it is easy to take out the dental prosthesis from the casting mold when manufacturing the dental prosthesis using the casting mold.
The three-dimensional object of the disclosure preferably includes a cured article (that is, a stereolithographic object) by stereolithography.
The method for manufacturing a cured article (for example, stereolithographic object) is as described above.
Preferable aspects of the three-dimensional object include a casting mold, and more specifically, a casting mold used for manufacturing a dental prosthesis.

[Method for Manufacturing Cured article]

The method for manufacturing a cured article of the disclosure includes a step of polymerizing a curable composition in the above-mentioned casting mold. For example, a cured article may be produced by producing a casting mold using the photocurable composition of the disclosure described above, injecting the curable composition into the produced casting mold, and polymerizing the injected curable composition. The curable composition to be injected into the casting mold is not particularly limited as long as the curable composition contains a polymerizable component that is polymerized by heat, light, or the like. For example, when an artificial tooth is arranged in a casting mold to produce a dental prosthesis such as a denture with a base, a conventionally known curable composition for producing a denture base may be injected into the casting mold to cure the curable composition for producing a denture base after injection.

[Method for Manufacturing Denture with Base]

A method for manufacturing a denture with a base according to the disclosure includes: a step of curing the photocurable composition by stereolithography to produce a casting mold used for manufacturing a dental prosthesis; and a step of polymerizing a curable composition in the casting mold to manufacture the dental prosthesis.
**In** the method for manufacturing a dental prosthesis according to the disclosure, a dental prosthesis is manufactured through two steps, namely a step of producing a casting mold and a step of manufacturing a dental prosthesis, and it is possible to manufacture a denture with a base by a method which is simpler than a method for producing a dental prosthesis using a conventional casting mold.
The step of producing a casting mold preferably includes: for example, a step of acquiring three-dimensional impression data in the oral cavity of the user of the dental prosthesis; a step of acquiring casting mold data from the acquired three-dimensional impression data; and a step of curing the photocurable composition by stereolithography based on the acquired casting mold data.
The step of manufacturing a dental prosthesis may be a step of manufacturing a denture with a base. It is preferable that the step of manufacturing a denture with a base includes: a step of arranging artificial teeth in a casting mold; a step of injecting a curable composition for producing a denture base into the casting mold in a state where the artificial teeth are arranged in the casting mold; a step of curing the curable composition after injection; and a step of removing the manufactured denture with a base from the casting mold.
**In the** method for manufacturing a dental prosthesis according to the disclosure, the photocurable composition used for producing a casting mold is not particularly limited. For example, from the viewpoint of suppressing deformation of the casting mold in the step of curing the curable composition after injection and from the viewpoint of suppressing damage to a dental prosthesis, a casting mold, or the like in the step of taking out a dental prosthesis manufactured from the casting mold, it is preferable to use the photocurable composition of the disclosure described above as a photocurable

composition to be used for producing the casting mold.

EXAMPLES

Hereinafter, Examples of the disclosure will be described, but the disclosure is not limited to the following Examples.

(Examples 1 to 17 and Comparative Examples 1 to 5)

<Preparation of Photocurable Composition>

Each component shown in Tables 1 to 5 was mixed to obtain a photocurable composition. Table 1 shows details of each component, and Tables 2 to 5 show mixing ratios of each component.

<Measurement and Evaluation>

Using the obtained photocurable composition, the following measurement and evaluation were performed.
The results are shown in Tables 2 to 5.

(Releasability from Denture)

The obtained photocurable composition was irradiated with visible light having a wavelength of 405 nm at an irradiation amount of 11 mJ/cm$^2$ to form a cured layer A1 having a thickness of 50 $\mu$m, the cured layer A1 was layered in the thickness direction to form a three-dimensional object A1 illustrated in Fig. 1, and a test piece A1 illustrated in Fig. 1 was produced by stereolithography under the condition of irradiating the shaped object A1 with ultraviolet light having a wavelength of 365 nm at an irradiation amount of 3 J/cm$^2$.
As shown in Fig. 1, the test piece A1 has a shape in which L is 24 mm, L' is 20 mm, W is 2 mm, H is 5 mm, H' is 3 mm, D is 14 mm, and D' is 10 mm. PalaXtreme (registered trademark, manufactured by Kulzer), which is a (meth)acrylate polymer powder for manufacturing a denture base and a (meth)acrylate monomer solution, are mixed at a ratio of 10 g of powder:6 g of liquid, and 30 seconds after the mixing, the obtained mixture is filled in a space composed of L' (20 mm) x D' (10 mm) x H' (3 mm) of the manufactured test piece A1, and polymerized at 55°C and a pressure of 2 bar for 30 minutes. Thereafter, the test piece A1 is removed to be peeled off from the denture base polymer. Thereafter, the surface of the denture base polymer that was in contact with the test piece A1 was observed with a 3D shape measuring instrument (VR-3200 manufactured by Keyence Corporation), the area value of the test piece A1 adhered to the denture base polymer after removal was calculated, and the adhesion rate to the surface (200 mm$^2$) including L' and D' was calculated (Tables 2 to 5). The smaller the adhesion rate, the better the releasability. The case where there was no surface adhesion was evaluated as "A", the case where the surface adhesion was less than 5% was evaluated as "B", and the case where the surface adhesion was 5% or more was evaluated as "C". The results are shown in Tables 2 to 5.

(Toughness during Mold Release)

The obtained photocurable composition was irradiated with visible light having a wavelength of 405 nm at an irradiation amount of 11 mJ/cm$^2$ to form the cured layer A1 having a thickness of 50 $\mu$m, the cured layer A1 was layered in the thickness direction to form the three-dimensional object A1 illustrated in Fig. 1, and the test piece A1 illustrated in Fig. 1 was produced by stereolithography under the condition of irradiating the shaped object A1 with ultraviolet light having a wavelength of 365 nm at an irradiation amount of 3 J/cm$^2$. The size of the test piece A1 used for the toughness evaluation is the same as the size of the test piece A1 used for the releasability evaluation described above.
A (meth)acrylate polymer powder for manufacturing a denture base and PalaXtreme (registered trademark, manufactured by Kulzer), which is a (meth)acrylate monomer solution, are mixed at a ratio of 10 g of powder:6 g of liquid, and 30 seconds after the mixing, the obtained mixture is filled in a space composed of L' (20 mm) x D' (10 mm) x H' (3 mm) of the manufactured test piece A1, and polymerized at 55°C and a pressure of 2 bar for 30 minutes. Thereafter, the test piece A1 is removed to be peeled off from the denture base polymer. The appearance of the test piece A1 after removal was observed, and the case where there was no breakage was evaluated as "A", and the case where there was breakage was evaluated as "B". The results are shown in Tables 2 to 5.

(Surface Roughness)

The obtained photocurable composition was shaped to "width of 20 mm x length 20 mm x thickness of 10 mm"

using a 3D printer (Cara Print 4.0 manufactured by Kulzer) under the conditions of a wavelength of visible light of 405 nm and an illuminance of visible light of 8.0 $mJ/cm^2$ to obtain a shaped object (layering width: 50 μm). In addition, at the time of layering, layering was performed in the thickness direction, and shaping was performed.

The obtained shaped object was irradiated with an ultraviolet ray having a wavelength of 365 nm under a condition of 10 $J/cm^2$ to finally cure the shaped object, thereby obtaining a stereolithographic object.

For the obtained stereolithographic object (hereinafter referred to as "test piece"), the surface roughness (Rz) of the test piece in the layering surface direction (surface having a width of 20 mm and a length of 20 mm) and in the layering height direction (surface having a width of 20 mm and a thickness of 10 mm) was measured using a 3D shape measuring instrument (VR-3200 manufactured by Keyence Corporation). The smaller the surface roughness Rz is, the better the surface smoothness is. Evaluation was performed based on the following criteria. The results are shown in Tables 2 to 5.

A:

$$Rz < 30\ \mu m$$

B:

$$30\ \mu m \leq Rz < 50\ \mu m$$

C:

$$50\ \mu m \leq Rz < 70\ \mu m$$

D:

$$70\ \mu m \leq Rz$$

(Evaluation of Appearance of Molded Object)

The obtained photocurable composition was irradiated with visible light having a wavelength of 405 nm at an irradiation amount of 11 $mJ/cm^2$ to form the cured layer A1 having a thickness of 50 μm, the cured layer A1 was layered in the thickness direction to form a three-dimensional object A1 illustrated in Fig. 1, and the test piece A1 illustrated in Fig. 1 was produced by stereolithography under the condition of irradiating the shaped object A1 with ultraviolet light having a wavelength of 365 nm at an irradiation amount of 3 $J/cm^2$. The test piece A1 illustrated in Fig. 1 was produced. The size of the test piece A1 used for the appearance evaluation is the same as the size of the test piece A1 used for the releasability evaluation described above.

A (meth)acrylate polymer powder for manufacturing a denture base and PalaXtreme (registered trademark, manufactured by Kulzer), which is a (meth)acrylate monomer solution, are mixed at a ratio of 10 g of powder:6 g of liquid, and 30 seconds after the mixing, the obtained mixture is filled in a space composed of L' (20 mm) × D' (10 mm) × H' (3 mm) of the manufactured test piece A1, and polymerized at 55°C and a pressure of 2 bar for 30 minutes. Thereafter, the test piece A1 is removed to be peeled off from the denture base polymer. Thereafter, the surface of the denture base polymer, which was in contact with the test piece A1, was visually observed and determined based on the following criteria. The results are shown in Tables 2 to 5.

A: The surface was smooth with no roughness.
B: The surface exhibited unevenness in appearance due to surface roughness.

[Table 1]

| | Component | Name | SP value $(cal/cm^3)^{1/2}$ | Cohesive energy density (cal/mol) | Mw | Molecular weight |
|---|---|---|---|---|---|---|
| Composition | Mono(meth)acrylic monomers (A-2) | PO-A | 10.12 | 17,150 | 192.21 | 192.21 |
| | | PO | 10.02 | 18,275 | 206.24 | 206.24 |
| | | P2H-A | 9.99 | 20,310 | 236.27 | 236.27 |
| | | M-600A | 11.93 | 25,090 | 222.24 | 222.24 |
| | | POB-A | 10.29 | 23,600 | 254.29 | 254.29 |
| | | A-LEN-10 | 10.19 | 24,780 | 268.31 | 268.31 |
| | Di(meth)acrylic monomers (A-1) | SUA-001 | 10.92 | 42,775 | 442.51 | 442.51 |
| | | AH-600 | 11.53 | 60,620 | 612.68 | 612.68 |
| | | SUA-002 | 10.81 | 91,840 | 951.16 | 700 to 1700 |
| | | SUA-003 | 10.11 | 143,330 | 1632.21 | 1000 to 3000 |
| | | SUA-004 | 8.71 | 297,595 | 4702.34 | 3500 to 7000 |
| | (Meth)acrylic monomers (B) | SiA-001 | 8.48 | 30,580 | 432.74 | 432.74 |
| | | SiA-002 | 8.50 | 108,160 | 1656.66 | 1000 to 3000 |
| | | SiA-003 | 7.69 | 96,200 | 1693.35 | 1000 to 3000 |
| | Compounds (C) | Lauryl alcohol | 9.81 | 21,225 | 186.34 | 186.34 |
| | | Myristic acid | 9.26 | 21,885 | 228.38 | 228.38 |
| | | Ethyl laurate | 8.63 | 19,530 | 228.38 | 228.38 |
| | | Isopropyl myristate | 8.54 | 22,655 | 270.45 | 270.45 |
| | | Isopropyl palmitate | 8.54 | 25,015 | 298.50 | 298.50 |
| | | Monoglycerol stearate | 10.76 | 41,725 | 358.56 | 358.56 |
| | | Glyceryl trioleate | 8.94 | 74,015 | 885.45 | 885.45 |
| | | Diisostearyl malate | 9.22 | 57,620 | 639.06 | 639.06 |
| | | Polypropylene glycol | 9.80 | 39,240 | 482.66 | 482.66 |
| | | Triethyl citrate | 11.46 | 29,645 | 276.28 | 276.28 |
| | | Trimethylolpropane | 14.20 | 28,680 | 134.17 | 134.17 |
| | | 1-Hexanol | 10.68 | 14,145 | 102.16 | 102.16 |
| | Other compounds | Octadecafluorooctane | 5.68 | 8,160 | 438.06 | 438.06 |
| | | Sorbitol | 23.42 | 48,360 | 182.17 | 182.17 |
| | Photopolymerization initiator | Omnirad819 | - | - | 348.38 | 348.38 |

[Table 2]

| | Component | Name | Example 1 | Example 2 | Example 3 | Example 4 | Example 5 | Example 6 |
|---|---|---|---|---|---|---|---|---|
| Composition | Mono(meth)acrylic monomers (A-2) | PO-A | 60 | | | 40 | | |
| | | PO | | 65 | | | | |
| | | P2H-A | | | 60 | | | |
| | | M-600A | | | | 20 | | |
| | | POB-A | | | | | 65 | |
| | | A-LEN-10 | | | | | | 60 |
| | Di(meth)acrylic monomers (A-1) | SUA-001 | 20 | | | | | |
| | | AH-600 | | 20 | | | | |
| | | SUA-002 | | | 20 | | 20 | |
| | | SUA-003 | | | | 20 | | |
| | | SUA-004 | | | | | | 20 |
| | (Meth)acrylic monomers (B) | SiA-001 | 10 | 10 | | 10 | | |
| | | SiA-002 | | | 10 | | 10 | |
| | | SiA-003 | | | | | | 10 |
| | Compounds (C) | Lauryl alcohol | 10 | | | | | |
| | | Myristic acid | | 5 | | | | |
| | | Ethyl laurate | | | 10 | | | |
| | | Isopropyl myristate | | | | | 5 | |
| | | Isopropyl palmitate | | | | | | 10 |
| | | Monoglycerol stearate | | | | 10 | | |
| | | Glyceryl trioleate | | | | | | |
| | | Diisostearyl malate | | | | | | |
| | | Polypropylene glycol | | | | | | |
| | | Triethyl citrate | | | | | | |
| | | Trimethylolpropane | | | | | | |
| | | 1-Hexanol | | | | | | |

(continued)

| | Component | Name | Example 1 | Example 2 | Example 3 | Example 4 | Example 5 | Example 6 |
|---|---|---|---|---|---|---|---|---|
| | Other compounds | Octadecafluorooctane | | | | | | |
| | | Sorbitol | | | | | | |
| | Photopolymerization initiator | Omnirad819 | 1 | 1 | 1 | 1 | 1 | 1 |
| Total | | | 101 | 101 | 101 | 101 | 101 | 101 |
| Siloxane bond concentration in the composition (mmol/g) | | | 0.686 | 0.686 | 0.502 | 0.686 | 0.502 | 1.169 |
| Aromatic ring concentration in all (meth)acrylic monomers (mol/g) | | | 0.0031 | 0.0038 | 0.0025 | 0.0030 | 0.0051 | 0.0044 |
| Average SP value of (meth)acrylic monomers $(cal/cm^3)^{1/2}$ | | | 10.12 | 10.18 | 10.01 | 10.34 | 10.21 | 9.58 |
| Average SP value of compound (C) $(cal/cm^3)^{1/2}$ | | | 9.81 | 9.26 | 8.63 | 10.76 | 8.54 | 8.54 |
| \|(Average SP value of (meth)acrylic monomers) - (Average SP value of compound (C))\| $(cal/cm^3)^{1/2}$ | | | 0.31 | 0.92 | 1.38 | (0.42) | 1.67 | 1.04 |
| Standard deviation of SP values among (meth)acrylic monomer components $(cal/cm^3)^{1/2}$ | | | 0.66 | 0.84 | 0.63 | 0.99 | 0.62 | 0.90 |
| Standard deviation of SP values of compound (C) $(cal/cm^3)^{1/2}$ | | | 0.00 | 0.00 | 0.00 | 0.00 | 0.00 | 0.00 |
| Average SP value between (meth)acrylic monomers and compound (C) $(cal/cm^3)^{1/2}$ | | | 10.09 | 10.13 | 9.87 | 10.38 | 10.13 | 9.48 |
| Standard deviation of SP value between (meth)acrylic monomers and compound (C) $(cal/cm^3)^{1/2}$ | | | 0.64 | 0.84 | 0.73 | 0.95 | 0.71 | 0.91 |
| Releasability with respect to dentures A: No surface adhesion, B: Surface adhesion < 5%, C: Surface adhesion ≥ 5% | | | A | A | A | A | A | A |
| Toughness at the time of mold release A: No breakage, B: Breakage occurred | | | A | A | A | A | A | A |
| Surface roughness of shaped object in layering surface direction (Rz) A: Rz < 30 μm, B: 30 μm ≤ Rz < 50 μm, C: 50 μm ≤ Rz < 70 μm, D: Rz ≥ 70 μm | | | A | A | A | A | A | A |
| Surface roughness of shaped object in layering height direction (Rz) A: Rz < 30 μm, B: 30 μm ≤ Rz < 50 μm, C: 50 μm ≤ Rz < 70 μm, D: Rz ≥ 70 μm | | | A | A | A | A | A | A |
| Appearance evaluation of molded object A: Surface was smooth with no roughness, B: Appearance showed unevenness due to surface roughness | | | A | A | A | A | A | A |

[Table 3]

| | Component | Name | Example 7 | Example 8 | Example 9 | Example 10 | Example 11 | Example 12 |
|---|---|---|---|---|---|---|---|---|
| Composition | Mono(meth)acrylic | PO-A | | | | | 30 | |
| | monomers (A-2) | PO | | | | | | |
| | | P2H-A | 65 | | | 65 | | |
| | | M-600A | | | | | | |
| | | POB-A | | | 65 | | | 60 |
| | | A-LEN-10 | | 60 | | | 30 | |
| | Di(meth)acrylic monomers (A-1) | SUA-001 | | | | | | |
| | | AH-600 | 20 | | | | 20 | 10 |
| | | SUA-002 | | | | 20 | | 10 |
| | | SUA-003 | | 20 | | | | |
| | | SUA-004 | | | 10 | | | |
| | (Meth)acrylic monomers (B) | SiA-001 | | | | | 10 | |
| | | SiA-002 | | 10 | | | | 10 |
| | | SiA-003 | 10 | | 15 | 10 | | |
| | Compounds (C) | Lauryl alcohol | | | | | 10 | |
| | | Myristic acid | | | | | | |
| | | Ethyl laurate | | | | | | |
| | | Isopropyl myristate | | | | | | |
| | | Isopropyl palmitate | | | | | | 10 |
| | | Monoglycerol stearate | 5 | | | | | |
| | | Glyceryl trioleate | | 10 | | | | |
| | | Diisostearyl malate | | | 10 | | | |
| | | Polypropylene glycol | | | | 5 | | |
| | | Triethyl citrate | | | | | | |
| | | Trimethylolpropane | | | | | | |
| | | 1-Hexanol | | | | | | |

(continued)

| | Component | Name | Example 7 | Example 8 | Example 9 | Example 10 | Example 11 | Example 12 |
|---|---|---|---|---|---|---|---|---|
| | Other compounds | Octadecafluorooctane | | | | | | |
| | | Sorbitol | | | | | | |
| | Photopolymerization initiator | Omnirad819 | 1 | 1 | 1 | 1 | 1 | 1 |
| Total | | | 101 | 101 | 101 | 101 | 101 | 101 |
| Siloxane bond concentration in the composition (mmol/g) | | | 1.169 | 0.502 | 1.754 | 1.169 | 0.686 | 0.502 |
| Aromatic ring concentration in all (meth)acrylic monomers (mol/g) | | | 0.0034 | 0.0044 | 0.0051 | 0.0027 | 0.0044 | 0.0050 |
| Average SP value of (meth)acrylic monomers $(cal/cm^3)^{1/2}$ | | | 10.07 | 9.98 | 9.68 | 9.92 | 10.27 | 10.29 |
| Average SP value of compound (C) $(cal/cm^3)^{1/2}$ | | | 10.76 | 8.94 | 9.22 | 9.80 | 9.81 | 8.54 |
| \|(Average SP value of (meth)acrylic monomers) - (Average SP value of compound (C))\| $(cal/cm^3)^{1/2}$ | | | (0.69) | 1.04 | 0.46 | 0.12 | 0.46 | 1.75 |
| Standard deviation of SP values among (meth)acrylic monomer components $(cal/cm^3)^{1/2}$ | | | 1.02 | 0.53 | 1.02 | 0.83 | 0.85 | 0.75 |
| Standard deviation of SP values of compound (C) $(cal/cm^3)^{1/2}$ | | | 0.00 | 0.00 | 0.00 | 0.00 | 0.00 | 0.00 |
| Average SP value between (meth)acrylic monomers and compound (C) $(cal/cm^3)^{1/2}$ | | | 10.11 | 9.88 | 9.64 | 9.91 | 10.23 | 10.11 |
| Standard deviation of SP value between (meth)acrylic monomers and compound (C) $(cal/cm^3)^{1/2}$ | | | 1.01 | 0.59 | 0.97 | 0.81 | 0.82 | 0.88 |
| Releasability with respect to dentures<br>A: No surface adhesion, B: Surface adhesion < 5%, C: Surface adhesion ≥ 5% | | | B | A | A | A | A | A |
| Toughness at the time of mold release<br>A: No breakage, B: Breakage occurred | | | A | A | A | A | A | A |
| Surface roughness of shaped object in layering surface direction (Rz)<br>A: Rz < 30 μm, B: 30 μm ≤ Rz < 50 μm, C: 50 μm ≤ Rz < 70 μm, D: Rz ≥ 70 μm | | | B | A | A | A | A | A |
| Surface roughness of shaped object in layering height direction (Rz)<br>A: Rz < 30 μm, B: 30 μm ≤ Rz < 50 μm, C: 50 μm ≤ Rz < 70 μm, D: Rz ≥ 70 μm | | | B | A | A | A | A | A |
| Appearance evaluation of molded object<br>A: Surface was smooth with no roughness, B: Appearance showed unevenness due to surface roughness | | | A | A | A | A | A | A |

[Table 4]

| | Component | Name | Example 13 | Example 14 | Example 15 | Example 16 | Example 17 |
|---|---|---|---|---|---|---|---|
| Composition | Mono(meth)acrylic monomers (A-2) | PO-A | | | 60 | 70 | 60 |
| | | PO | | | | | |
| | | P2H-A | | 40 | | | |
| | | M-600A | | | | | |
| | | POB-A | | | | | |
| | | A-LEN-10 | 60 | | | | |
| | Di(meth)acrylic monomers (A-1) | SUA-001 | | | | | |
| | | AH-600 | | | | 20 | |
| | | SUA-002 | | 40 | 20 | | |
| | | SUA-003 | 10 | | | | 25 |
| | | SUA-004 | | | | | |
| | (Meth)acrylic monomers (B) | SiA-001 | 10 | | | 5 | |
| | | SiA-002 | | 10 | | | 10 |
| | | SiA-003 | 10 | | 10 | | |
| | Compounds (C) | Lauryl alcohol | 5 | | | | |
| | | Myristic acid | | | | | |
| | | Ethyl laurate | 5 | | | | |
| | | Isopropyl myristate | | 10 | | | |
| | | Isopropyl palmitate | | | | | |
| | | Monoglycerol stearate | | | | | |
| | | Glyceryl trioleate | | | | | |
| | | Diisostearyl malate | | | | | |
| | | Polypropylene glycol | | | | | |
| | | Triethyl citrate | | | 10 | | |
| | | Trimethylolpropane | | | | 5 | |
| | | 1-Hexanol | | | | | 5 |

(continued)

| | Component | Name | Example 13 | Example 14 | Example 15 | Example 16 | Example 17 |
|---|---|---|---|---|---|---|---|
| | Other compounds | Octadecafluorooctane | | | | | |
| | | Sorbitol | | | | | |
| | Photopolymerization initiator | Omnirad819 | 1 | 1 | 1 | 1 | 1 |
| Total | | | 101 | 101 | 101 | 101 | 101 |
| Siloxane bond concentration in the composition (mmol/g) | | | 1.856 | 0.502 | 1.169 | 0.343 | 0.502 |
| Aromatic ring concentration in all (meth)acrylic monomers (mol/g) | | | 0.0044 | 0.0017 | 0.0031 | 0.0043 | 0.0031 |
| Average SP value of (meth)acrylic monomers $(cal/cm^3)^{1/2}$ | | | 9.71 | 10.19 | 10.00 | 10.33 | 9.95 |
| Average SP value of compound (C) $(cal/cm^3)^{1/2}$ | | | 9.22 | 8.54 | 11.46 | 14.20 | 10.68 |
| [(Average SP value of (meth)acrylic monomers) - (Average SP value of compound (C))\| $(cal/cm^3)^{1/2}$ | | | 0.49 | 1.65 | (1.46) | (3.87) | (0.73) |
| Standard deviation of SP values among (meth)acrylic monomer components $(cal/cm^3)^{1/2}$ | | | 0.89 | 0.71 | 0.87 | 0.72 | 0.50 |
| Standard deviation of SP values of compound (C) $(cal/cm^3)^{1/2}$ | | | 0.59 | 0.00 | 0.00 | 0.00 | 0.00 |
| Average SP value between (meth)acrylic monomers and compound (C) (cal/cm3)1/2 | | | 9.66 | 10.02 | 10.15 | 10.52 | 9.98 |
| Standard deviation of SP value between (meth)acrylic monomers and compound (C) $(cal/cm^3)^{1/2}$ | | | 0.88 | 0.84 | 0.93 | 1.10 | 0.51 |
| Releasability with respect to dentures<br>A: No surface adhesion, B: Surface adhesion < 5%, C: Surface adhesion ≥ 5% | | | A | A | B | B | A |
| Toughness at the time of mold release<br>A: No breakage, B: Breakage occurred | | | A | A | A | A | A |
| Surface roughness of shaped object in layering surface direction (Rz)<br>A: Rz < 30 μm, B: 30 μm ≤ Rz < 50 μm, C: 50 μm ≤ Rz < 70 μm, D: Rz ≥ 70 μm | | | A | A | B | B | A |
| Surface roughness of shaped object in layering height direction (Rz)<br>A: Rz < 30 μm, B: 30 μm ≤ Rz < 50 μm, C: 50 μm ≤ Rz < 70 μm, D: Rz ≥ 70 μm | | | A | A | B | B | A |
| Appearance evaluation of molded object<br>A: Surface was smooth with no roughness, B: Appearance showed unevenness due to surface roughness | | | A | A | A | A | A |

[Table 5]

| | Component | Name | Comparative Example 1 | Comparative Example 2 | Comparative Example 3 | Comparative Example 4 | Comparative Example 5 |
|---|---|---|---|---|---|---|---|
| Composition | Mono(meth)acrylic monomers (A-2) | PO-A | 80 | 75 | 70 | 60 | 60 |
| | | PO | | | | | |
| | | P2H-A | | | | | |
| | | M-600A | | | | | |
| | | POB-A | | | | | |
| | | A-LEN-10 | | | | | |
| | Di(meth)acrylic monomers (A-1) | SUA-001 | 20 | 20 | 20 | 20 | 20 |
| | | AH-600 | | | | | |
| | | SUA-002 | | | | | |
| | | SUA-003 | | | | | |
| | | SUA-004 | | | | | |
| | (Meth)acrylic monomers (B) | SiA-001 | | | 10 | 10 | 10 |
| | | SiA-002 | | | | | |
| | | SiA-003 | | | | | |
| | Compounds (C) | Lauryl alcohol | | 5 | | | |
| | | Myristic acid | | | | | |
| | | Ethyl laurate | | | | | |
| | | Isopropyl myristate | | | | | |
| | | Isopropyl palmitate | | | | | |
| | | Monoglycerol stearate | | | | | |
| | | Glyceryl trioleate | | | | | |
| | | Diisostearyl malate | | | | | |
| | | Polypropylene glycol | | | | | |
| | | Triethyl citrate | | | | | |
| | | Trimethylolpropane | | | | | |
| | | 1-Hexanol | | | | | |

(continued)

| | Component | Name | Comparative Example 1 | Comparative Example 2 | Comparative Example 3 | Comparative Example 4 | Comparative Example 5 |
|---|---|---|---|---|---|---|---|
| | Other compounds | Octadecafluorooctane | | | | 10 | |
| | | Sorbitol | | | | | 10 |
| | Photopolymerization initiator | Omnirad819 | 1 | 1 | 1 | 1 | 1 |
| Total | | | 101 | 101 | 101 | 101 | 101 |
| Siloxane bond concentration in the composition (mmol/g) | | | 0.000 | 0.000 | 0.686 | 0.686 | 0.686 |
| Aromatic ring concentration in all (meth)acrylic monomers (mol/g) | | | 0.0041 | 0.0039 | 0.0036 | 0.0031 | 0.0031 |
| Average SP value of (meth)acrylic monomers $(cal/cm^3)^{1/2}$ | | | 10.28 | 10.29 | 10.12 | 10.12 | 10.12 |
| Average SP value of compound (C) $(cal/cm^3)^{1/2}$ | | | - | 9.81 | - | 5.68 | 23.42 |
| \|(Average SP value of (meth)acrylic monomers) - (Average SP value of compound (C))\| $(cal/cm^3)^{1/2}$ | | | - | 0.48 | - | 4.44 | (13.30) |
| Standard deviation of SP values among (meth)acrylic monomer components $(cal/cm^3)^{1/2}$ | | | 0.32 | 0.33 | 0.63 | 0.66 | 0.66 |
| Standard deviation of SP values of compound (C) $(cal/cm^3)^{1/2}$ | | | - | 0.00 | - | 0.00 | 0.00 |
| Average SP value between (meth)acrylic monomers and compound (C) $(cal/cm^3)^{1/2}$ | | | 10.28 | 10.26 | 10.12 | 9.67 | 11.45 |
| Standard deviation of SP value between (meth)acrylic monomers and compound (C) $(cal/cm^3)^{1/2}$ | | | 0.32 | 0.33 | 0.63 | 1.47 | 4.04 |
| Releasability with respect to dentures A: No surface adhesion, B: Surface adhesion < 5%, C: Surface adhesion ≥ 5% | | | C | C | - | - | - |
| Toughness at the time of mold release A: No breakage, B: Breakage occurred | | | B | B | - | - | - |
| Surface roughness of shaped object in layering surface direction (Rz) A: Rz < 30 μm, B: 30 μm ≤ Rz < 50 μm, C: 50 μm ≤ Rz < 70 μm, D: Rz ≥ 70 μm | | | C | C | D | D | D |
| Surface roughness of shaped object in layering height direction (Rz) A: Rz < 30 μm, B: 30 μm ≤ Rz < 50 μm, C: 50 μm ≤ Rz < 70 μm, D: Rz ≥ 70 μm | | | C | C | D | D | D |
| Appearance evaluation of molded object A: Surface was smooth with no roughness, B: Appearance showed unevenness due to surface roughness | | | B | B | B | B | B |

In Tables 2 to 5, the number in the "composition" field in each Example and each Comparative Example means parts by mass, and the blank means that the corresponding component is not contained. The compound (C) and the like in Table 5 mean the compound (C) or other compounds.

<Mono(Meth)Acrylic Monomer (A-2)>

In Tables 1 to 5, each of the structures of the compounds classified into the mono(meth)acrylic monomer (A-2) is as follows.

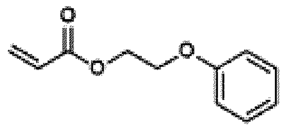

PO-A

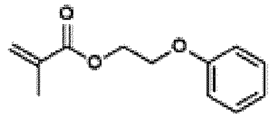

PO

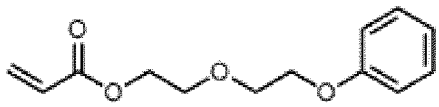

P2H-A

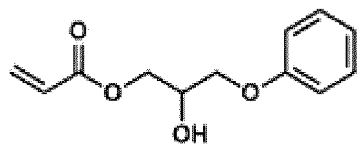

M-600A

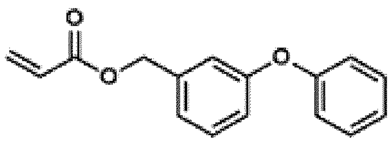

POB-A

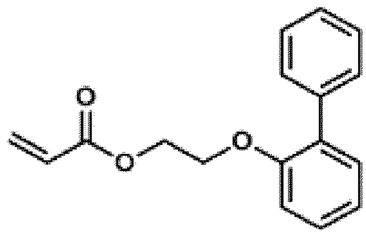

A-LEN-10

PO-A: Phenoxyethyl acrylate (Kyoeisha Chemical Co., Ltd.)
PO: Phenoxyethyl methacrylate (Kyoeisha Chemical Co., Ltd.)
P2H-A: Phenoxydiethylene glycol acrylate (Kyoeisha Chemical Co., Ltd.)
M-600A: 2-Hydroxy-3-phenoxypropyl acrylate (Kyoeisha Chemical Co., Ltd.)
POB-A: m-Phenoxybenzyl acrylate (Kyoeisha Chemical Co., Ltd.)
A-LEN-10: Ethoxylated o-phenylphenol acrylate (Shin-Nakamura Chemical Co., Ltd.)

<Di(Meth)Acrylic Monomer (A-1)>

In Tables 1 to 5, each of the structures of the compounds classified into the di(meth)acrylic monomer (A-1) is as follows.

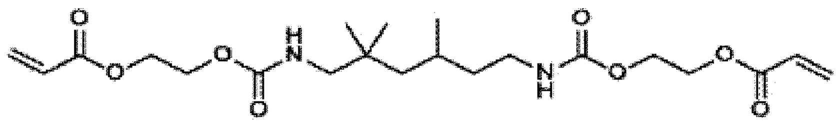

SUA-1

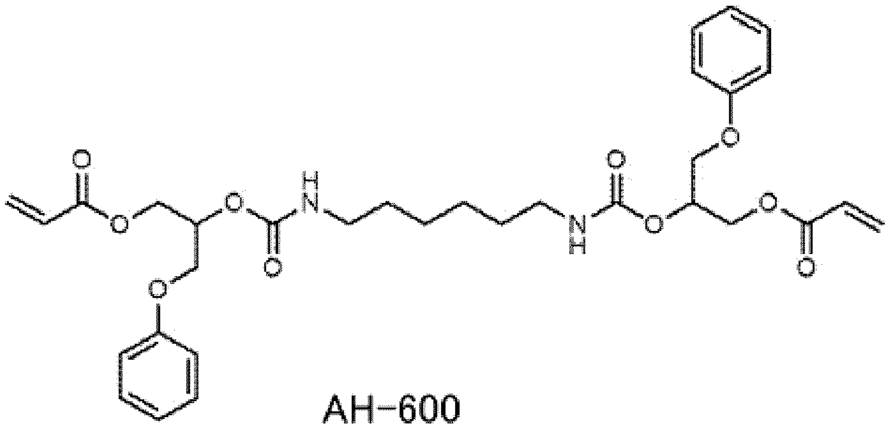

AH-600

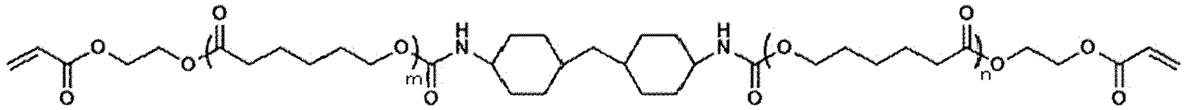

SA-002

m + n ≒ 4

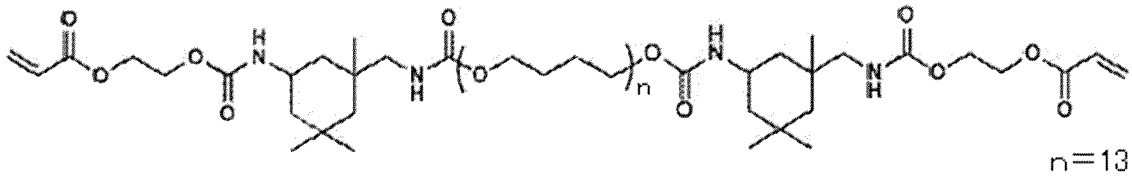

n=13

SUA-003

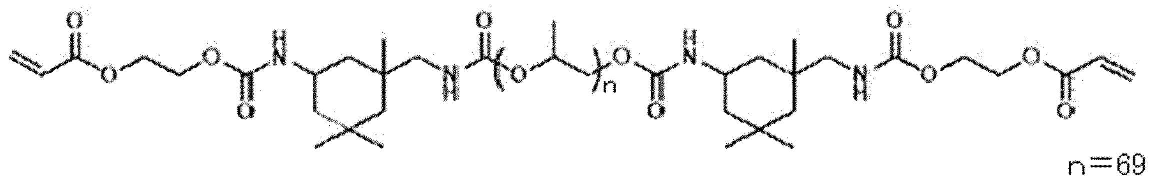

n=69

SUA-004

SUA-001: A compound manufactured by the method described in the following Manufacturing Example 1A
AH-600: Difunctional urethane acrylate (manufactured by Kyoeisha Chemical Co., Ltd.)
SUA-002: A compound manufactured by the method described in the following Manufacturing Example 2A
SUA-003: A compound manufactured by the method described in the following Manufacturing Example 3A
SUA-004: A compound manufactured by the method described in the following Manufacturing Example 4A

[Manufacturing Example 1A: Manufacturing of SUA-1]

Into a 1-liter four-neck flask equipped with a thoroughly dried stirring blade and a thermometer, 372 g (3.20 mol) of HEA (2-hydroxyethyl acrylate), 0.71 g (0.1% by mass with respect to the total mass of HEA and TMHDI) of DBTDL (dibutyltin dilaurate), and 0.35 g (0.05% by mass with respect to the total mass of HEA and TMHDI) of MEHQ (4-methoxyphenol) were added, stirred until the mixture became uniform, and then the temperature was raised to 60°C. Subsequently, 337 g (1.60 mol) of TMHDI (trimethylhexamethylene diisocyanate) was added dropwise over 1 hour. Since the internal temperature increased due to the reaction heat during the dropwise addition, the rate of dropwise addition was controlled such that the internal temperature was 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 680 g of difunctional urethane acrylate (SUA-1). The viscosity at 25°C was 7,100 mPa·s.

[Manufacturing Example 2A: Manufacturing of SUA-2]

Into a 1-liter four-neck flask equipped with a thoroughly dried stirring blade and a thermometer, 344 g (1.00 mol) of caprolactone-modified 2-hydroxyethyl acrylate (trade name "PLACCEL FA2D", manufactured by Daicel Corporation, average caprolactone addition number: 2), 131 g (0.50 mol) of dicyclohexylmethane-4,4'-diisocyanate, 0.84 g of DBTDL (dibutyltin dilaurate), and 0.42 g of MEHQ (4-methoxyphenol) were added, and the reaction was carried out at 80°C for 12 hours. At this time, the progress of the reaction was followed by HPLC analysis to confirm the end point of the reaction. The product was discharged from the reactor to obtain 451 g of difunctional urethane acrylate (SUA-2). The viscosity at 25°C was 12,000 mPa·s.

[Manufacturing Example 3A: Manufacturing of SUA-3]

Into a 1-liter four-neck flask equipped with a thoroughly dried stirring blade and a thermometer, 222 g (1.00 mol) of IPDI (isophorone diisocyanate), 0.84 g (0.1% by mass with respect to the total mass of **IPDI,** PTMG1000, and HEA) of DBTDL (dibutyltin dilaurate), and 0.42 g (0.05% by mass with respect to the total mass of IPDI, PEG-1000, and HEA) of MEHQ (4-methoxyphenol) were added, stirred until the mixture became uniform, and then the temperature was raised to 60°C. Subsequently, 500 g (0.50 mol) of PTMG1000 (molecular weight: 1,000, manufactured by Mitsubishi Chemical Corporation) was added dropwise over 1 hour. Since the internal temperature increased due to the reaction heat during the dropwise addition, the rate of dropwise addition was controlled such that the internal temperature was 80°C or less. After dropping the whole amount, the reaction was carried out for 5 hours while the reaction temperature was maintained at 80°C.
Subsequently, the internal temperature of the flask was maintained at 60°C, and 116 g (1.00 mol) of HEA (2-hydroxyethyl acrylate) added to another dropping funnel was added dropwise thereto over 1 hour. Since the internal temperature increased due to the reaction heat during the dropwise addition, the rate of dropwise addition was controlled such that the internal temperature was 80°C or less. After dropping the whole amount, the reaction was carried out for 5 hours while the reaction temperature was maintained at 80°C.
At this time, the progress of the reaction was followed by HPLC analysis to confirm the end point of the reaction. The product was discharged from the reactor to obtain 840 g of multifunctional urethane acrylate (SUA-3). The viscosity at 25°C was 63,000 mPa·s.

[Manufacturing Example 4A: Manufacturing of SUA-4]

Into a 1-liter four-neck flask equipped with a thoroughly dried stirring blade and a thermometer, 222 g (1.00 mol) of IPDI (isophorone diisocyanate), 2.34 g (0.1% by mass relative to the total mass of IPDI, polypropylene glycol 4000, and HEA) of DBTDL (dibutyltin dilaurate), and 1.17 g (0.05% by mass relative to the total mass of IPDI, polypropylene glycol 4000, and HEA) of MEHQ (4-methoxyphenol) were added, stirred until the mixture became uniform, and then the temperature was raised to 60°C. Subsequently, 2,000 g (0.50 mol) of polypropylene glycol 4000 (molecular weight: 4,000, manufactured by FUJIFILM Wako Pure Chemical Corporation) was added dropwise over 1 hour. Since the internal temperature increased due to the reaction heat during the dropwise addition, the rate of dropwise addition was controlled such that the internal temperature was 80°C or less. After dropping the whole amount, the reaction was carried out for 5 hours while the reaction temperature was maintained at 80°C.
Subsequently, the internal temperature of the flask was maintained at 60°C, and 116 g (1.00 mol) of HEA (2-hydroxyethyl acrylate) added to another dropping funnel was added dropwise thereto over 1 hour. Since the internal temperature increased due to the reaction heat during the dropwise addition, the rate of dropwise addition was controlled such that the internal temperature was 80°C or less. After dropping the whole amount, the reaction was carried out for 5 hours while the reaction temperature was maintained at 80°C.
At this time, the progress of the reaction was followed by HPLC analysis to confirm the end point of the reaction. The product was discharged from the reactor to obtain 2,410 g of multifunctional urethane acrylate (SUA-4). The viscosity at 25°C was 43,000 mPa·s.

<(Meth)Acrylic Monomer (B)>

In Tables 1 to 5, each of the structures of the compounds classified into the (meth)acrylic monomer (B) is as follows.

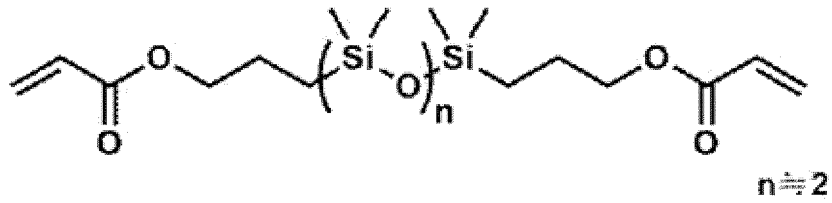

SiA-001

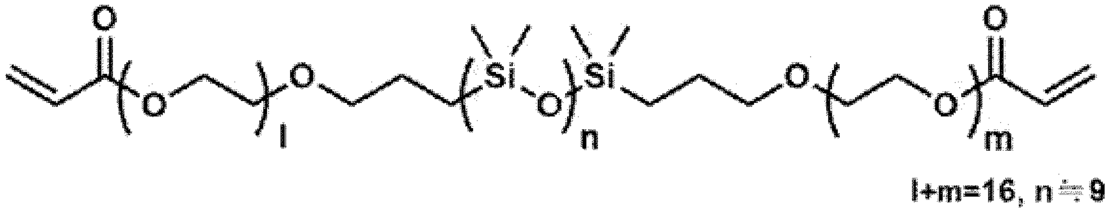

SiA-002

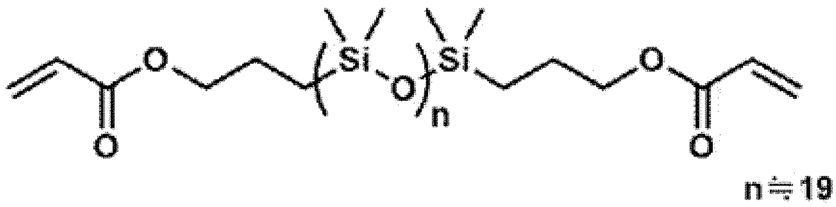

SiA-003

SiA-001: A compound manufactured by the method described in the following Manufacturing Example 1B
SiA-002: A compound manufactured by the method described in the following Manufacturing Example 2B
SiA-003: A compound manufactured by the method described in the following Manufacturing Example 3B

[Manufacturing Example 1B: Manufacturing of SiA-001]

Into a 1-liter four-neck flask equipped with a thoroughly dried stirring blade and a thermometer, 324 g (1.00 mol) of 3,3'-(1,1,3,3,5,5-hexamethyl-1,5-trisiloxanediyl)bis[1-propanol], 0.30 g of BHT, and 500 g of ethyl acetate were added, stirred until the mixture became uniform, and then the temperature was raised to 70°C. Subsequently, 181 g (2.00 mol) of acryloyl chloride was added dropwise over 1 hour. Since the internal temperature increased due to the reaction heat during the dropwise addition, the rate of dropwise addition was controlled such that the internal temperature was 70°C or less. After dropping the whole amount, the reaction was carried out for 5 hours while the reaction temperature was maintained at 70°C. Thereafter, the reaction solution was neutralized with a 10% NaOH aqueous solution, and then washed with 150 g of a 5% aqueous ammonium sulfate solution. Ethyl acetate was distilled under reduced pressure to obtain 440 g of a multifunctional (meth)acrylic monomer (SiA-001). The viscosity at 25°C was 110 mPa·s.

[Manufacturing Example 2B: Manufacturing of SiA-002]

Into a 1-liter four-neck flask equipped with a thoroughly dried stirring blade and a thermometer, 287 g (1.00 mol) of 1,3-bis(3-chloropropyl)-1,1,3,3-tetramethyldisiloxane, 0.30 g of BHT, and 500 g of toluene were added, stirred until the mixture became uniform, and then the temperature was raised to 60°C. Subsequently, 593 g (2.00 mol) of octamethylcyclotetrasiloxane was added dropwise over 1 hour. After dropping the whole amount, the reaction was carried out for 6 hours while the reaction temperature was maintained at 60°C. Thereafter, 740 g (2.00 mol) of octaethylene glycol was added to the reaction vessel, and the reaction was further carried out for 1 hour. Subsequently, 181 g (2.00 mol) of acryloyl chloride was added dropwise over 1 hour. Since the internal temperature increased due to the reaction heat during the dropwise addition, the rate of dropwise addition was controlled such that the internal temperature was 60°C or less. After

dropping the whole amount, the reaction was carried out for 5 hours while the reaction temperature was maintained at 60°C. Thereafter, the reaction solution was neutralized with a 10% NaOH aqueous solution, and then washed with 150 g of a 5% aqueous ammonium sulfate solution. Toluene was distilled under reduced pressure to obtain 440 g of a multifunctional (meth)acrylic monomer (SiA-002). The viscosity at 25°C was 310 mPa·s.

[Manufacturing Example 3B: Manufacturing of SiA-003]

Into a 1-liter four-neck flask equipped with a thoroughly dried stirring blade and a thermometer, 667 g (3.00 mol) of hexamethylcyclotrisiloxane, 100 mL of toluene, 200 mL of tetrahydrofuran, and 100 mL of hexane were added and stirred until the mixture became uniform. Thereafter, under ice cooling, 2 mL of butyllithium (approximately 15% hexane solution) was added dropwise over 1 hour. After dropping the whole amount, the reaction was carried out for 2 hours while the reaction temperature was maintained at room temperature. Subsequently, 207 g of 3-(chlorodimethylsilyl)propyl acrylate (1.00 mol), added to a separate dropping funnel, was added dropwise over 1 hour. After dropping the whole amount, the reaction was carried out for 24 hours while the reaction temperature was maintained at 30°C. At this time, the progress of the reaction was followed by HPLC analysis to confirm the end point of the reaction. Thereafter, the reaction solution was neutralized with a 10% NaOH aqueous solution, and then washed with 150 g of a 5% aqueous ammonium sulfate solution. The solvent was distilled under reduced pressure to obtain 812 g of a multifunctional (meth)acrylic monomer (SiA-003). The viscosity at 25°C was 280 mPa·s.

<Compound (C)>

In Tables 1 to 5, each of the structures of the compounds classified as the compound (C) is as follows.

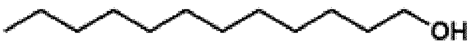

(C－1)

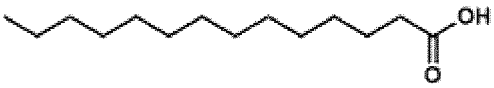

(C－2)

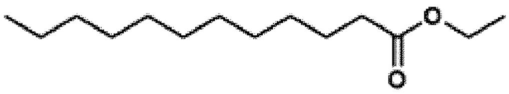

(C－3)

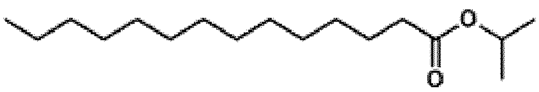

(C－4)

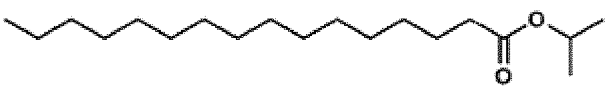

(C－5)

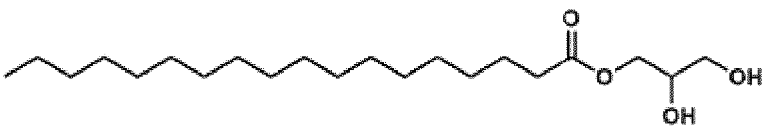

(C－6)

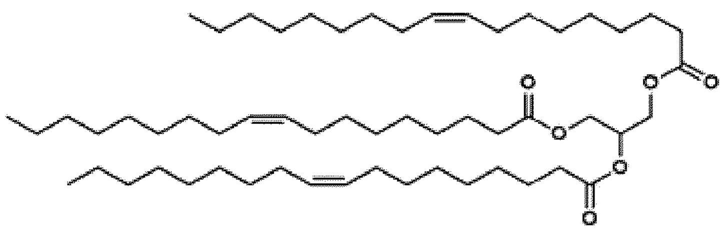

(C-7)

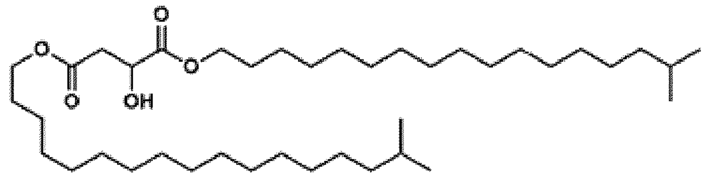

(C-8)

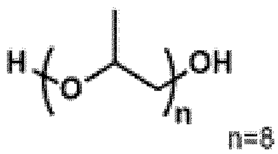

(C-9)

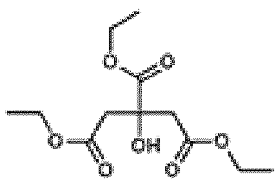

(C-10)

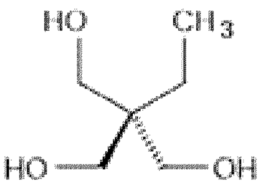

(C-11)

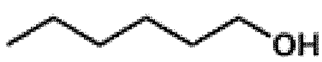

(C-12)

(C-1): Lauryl alcohol (FUJIFILM Wako Pure Chemical Corporation)
(C-2): Myristic acid (FUJIFILM Wako Pure Chemical Corporation)
(C-3): Ethyl laurate (FUJIFILM Wako Pure Chemical Corporation)
(C-4): Isopropyl myristate (FUJIFILM Wako Pure Chemical Corporation)
(C-5): Isopropyl palmitate (FUJIFILM Wako Pure Chemical Corporation)
(C-6): monoglycerol stearate (FUJIFILM Wako Pure Chemical Corporation)

(C-7): Glyceryl trioleate (Sigma-Aldrich)
(C-8): Diisostearyl malate (Kokyu Alcohol Kogyo Co., Ltd.)
(C-9): Polypropylene glycol (Sigma-Aldrich)
(C-10): Triethyl citrate (FUJIFILM Wako Pure Chemical Corporation)
(C-11): Trimethylolpropane (Tokyo Chemical Industry Co., Ltd.)
(C-12): 1-Hexanol (FUJIFILM Wako Pure Chemical Corporation)

<Other Compounds>

In Tables 1 to 5, each of the structures of the compounds classified as other compounds is as follows.

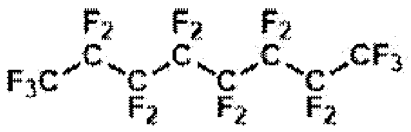

(1)

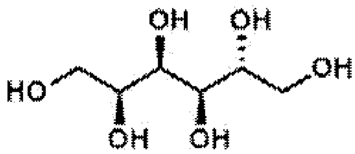

(2)

(1): Octafluorodecane (Sigma-Aldrich)
(2): Sorbitol (Tokyo Chemical Industry Co., Ltd.)

<Photopolymerization Initiator>

In Tables 1 to 5, the compounds classified as photopolymerization initiators are specifically the following compounds.

Acylphosphine oxide compound (Omnirad 819: "Omnirad 819" manufactured by IGM Resins B.V.)

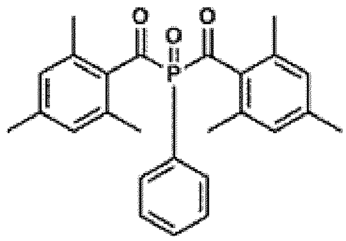

Omnirad819

As shown in Tables 2 to 5, it was possible to produce a three-dimensional object having suppressed surface roughness by using the photocurable composition of Examples 1 to 17.

On the other hand, the three-dimensional object obtained using the photocurable composition of Comparative Examples 1 to 5 was inferior in surface roughness to Example 1 to 20.

Furthermore, in Examples 1 to 17, evaluation of releasability, toughness, and appearance was also good.

The disclosure of Japanese Patent Application No. 2023-111074 filed on July 5, 2023 is incorporated herein by reference in its entirety.

All publications, patent applications, and technical standards mentioned in the present specification are incorporated in the present specification to the same extent as if each individual publication, patent application, and

technical standard were specifically and individually noted to be incorporated by reference.

## Claims

1. A photocurable composition, comprising:

   a (meth)acrylic monomer (A) having one or more (meth)acryloyl groups and not having a siloxane bond;
   a (meth)acrylic monomer (B) having two or more (meth)acryloyl groups and a siloxane bond;
   a compound (C) not having a (meth)acryloyl group and having a solubility parameter (SP value) of from 7.00 to 15.00 $(cal/cm^3)^{1/2}$; and
   a photopolymerization initiator.

2. The photocurable composition according to claim 1, wherein an absolute value of a difference between an average value of solubility parameters (SP values) of (meth)acrylic monomer components contained in the photocurable composition and an average value of the solubility parameter (SP value) of the compound (C) contained in the photocurable composition is 4.00 $(cal/cm^3)^{1\ 2}$ or less.

3. The photocurable composition according to claim 1, wherein a cohesive energy density (cal/mol) of the compound (C) is 10,000 or more.

4. The photocurable composition according to claim 1, wherein the (meth)acrylic monomer (A) contains:

   a di(meth)acrylic monomer (A-1) having at least one of a ring structure or a urethane bond, and two (meth)acryloyloxy groups, and not having a siloxane bond; and
   a mono(meth)acrylic monomer (A-2) having one (meth)acryloyloxy group and an aromatic ring, and not having a siloxane bond.

5. The photocurable composition according to claim 4, wherein a molecular weight of the di(meth)acrylic monomer (A-1) is from 400 to 8,000.

6. The photocurable composition according to claim 4, wherein a molecular weight of the mono(meth)acrylic monomer (A-2) is from 160 to 400.

7. The photocurable composition according to claim 1, wherein a molecular weight of the (meth)acrylic monomer (B) is from 400 to 5,000.

8. The photocurable composition according to claim 4, wherein a content of the di(meth)acrylic monomer (A-1) contained in the photocurable composition is from 3% by mass to 50% by mass with respect to a total content of (meth)acrylic monomer components and the compound (C) contained in the photocurable composition.

9. The photocurable composition according to claim 4, wherein a content of the mono(meth)acrylic monomer (A-2) contained in the photocurable composition is from 20% by mass to 85% by mass with respect to a total content of (meth)acrylic monomer components and the compound (C) contained in the photocurable composition.

10. The photocurable composition according to claim 1, wherein a content of the (meth)acrylic monomer (B) contained in the photocurable composition is from 1% by mass to 35% by mass with respect to a total content of (meth)acrylic monomer components and the compound (C) contained in the photocurable composition.

11. The photocurable composition according to claim 1, wherein a content of the compound (C) contained in the photocurable composition is from 1% by mass to 30% by mass with respect to a total content of (meth)acrylic monomer components and the compound (C) contained in the photocurable composition.

12. The photocurable composition according to claim 1, wherein a siloxane bond concentration in the photocurable composition is from 0.100 mmol/g to 3.000 mmol/g.

13. The photocurable composition according to claim 1, wherein an aromatic ring concentration in (meth)acrylic monomer components contained in the photocurable composition is from 0.0015 mol/g to 0.0070 mol/g.

**14.** The photocurable composition according to claim 1, which is a photocurable composition for stereolithography.

**15.** The photocurable composition according to claim 1, which is a photocurable composition used for manufacturing a casting mold by stereolithography.

**16.** A three-dimensional object, comprising a cured article of the photocurable composition according to any one of claims 1 to 15.

**17.** A casting mold, comprising the three-dimensional object according to claim 16.

**18.** The casting mold according to claim 17, used for manufacturing a dental prosthesis.

**19.** A method for manufacturing a cured article, comprising a step of polymerizing a curable composition in the casting mold according to claim 17.

**20.** A method for manufacturing a dental prosthesis, the method comprising:

a step of curing the photocurable composition according to any one of claims 1 to 15 by stereolithography to produce a casting mold used for manufacturing a dental prosthesis; and
a step of polymerizing a curable composition in the casting mold to manufacture the dental prosthesis.

Fig. 1

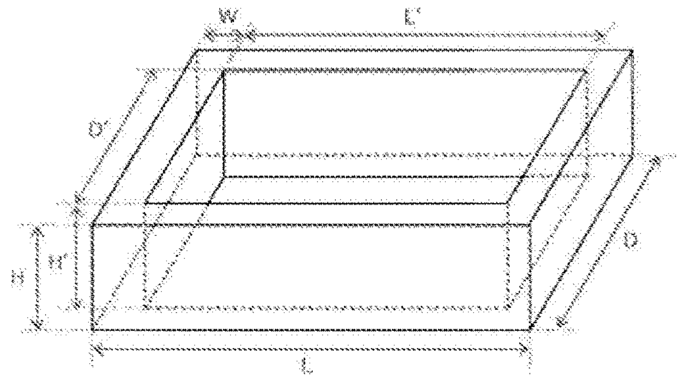

**INTERNATIONAL SEARCH REPORT**

International application No. **PCT/JP2024/024289**

**A. CLASSIFICATION OF SUBJECT MATTER**

***C08F 220/10***(2006.01)i; ***A61K 6/887***(2020.01)i; ***A61K 6/896***(2020.01)i; ***B33Y 70/00***(2020.01)i; ***B33Y 80/00***(2015.01)i; ***C08F 2/44***(2006.01)i; ***C08F 290/06***(2006.01)i
FI: C08F220/10; B33Y70/00; B33Y80/00; C08F2/44 Z; C08F290/06; A61K6/887; A61K6/896

According to International Patent Classification (IPC) or to both national classification and IPC

**B. FIELDS SEARCHED**

Minimum documentation searched (classification system followed by classification symbols)

C08F220/10; A61K6/887; A61K6/896; B33Y70/00; B33Y80/00; C08F2/44; C08F290/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2022/044117 A1 (MENICON CO., LTD.) 03 March 2022 (2022-03-03) claims, paragraphs [0121], [0124]-[0125] | 1, 3, 11, 14, 16-17, 19 |
| A | WO 2013/014733 A1 (MITSUI CHEMICALS, INC.) 31 January 2013 (2013-01-31) entire text | 1-20 |
| A | JP 2016-505525 A (DENTSPLY INTERNATIONAL INC.) 25 February 2016 (2016-02-25) entire text | 1-20 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 August 2024** | **20 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

## INTERNATIONAL SEARCH REPORT
**Information on patent family members**

International application No.
**PCT/JP2024/024289**

| Patent document cited in search report | Publication date (day/month/year) | Patent family member(s) | Publication date (day/month/year) |
|---|---|---|---|
| WO 2022/044117 A1 | 03 March 2022 | US 2024/0026145 A1<br>claims, paragraphs [0213]-[0258]<br>EP 4206797 A1<br>CN 115997158 A | |
| WO 2013/014733 A1 | 31 January 2013 | US 2014/0154451 A1<br>entire text<br>EP 2738188 A1<br>CN 103703036 A | |
| JP 2016-505525 A | 25 February 2016 | US 2014/0131908 A1<br>entire text<br>EP 2919705 A1<br>CN 104853693 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4160311 B
- JP 5111880 B
- JP 5235056 B
- JP 2023111074 A